# EUROPEAN PATENT SPECIFICATION

(11) **EP 3 456 815 B1**
(45) Date of publication and mention of the grant of the patent: **03.07.2024**
(21) Application number: 17796210.7
(22) Date of filing: 11.05.2017
(51) Int. Cl.: C12N 5/00, A61K 9/08, A61K 9/10, A61K 33/00

(54) **AQUEOUS SOLUTION CAPABLE OF BEING ADMINISTERED TO LIVING BODY, AND METHOD FOR PRODUCING SAME**
WÄSSRIGE LÖSUNG ZUR VERABREICHUNG AN EINEN LEBENDEN KÖRPER UND VERFAHREN ZUR HERSTELLUNG DAVON
SOLUTION AQUEUSE POUVANT ÊTRE ADMINISTRÉE À UN CORPS VIVANT ET PROCÉDÉ POUR SA PRODUCTION

(30) Priority: 13.05.2016 JP 2016096775
(43) Date of publication of application: 20.03.2019
(73) Proprietor: SIGMA TECHNOLOGY, Inc., Osaka City, Osaka (JP)
(72) Inventor: YAMANOUCHI, Dai, Madison, WI 53705 (US); TACHIBANA, Yoshiaki, Hitachinaka-shi Ibaraki 312-0011 (JP); TACHIBANA, Kosuke, Hitahcinaka-shi Ibaraki 312-0011 (JP)
(74) Representative: Dennemeyer & Associates S.A.
(86) International application number: PCT/JP2017/017779
(87) International publication number: WO 2017/195852

(56) References cited:
- EP-A1- 2 772 536
- EP-A1- 3 009 184
- EP-A1- 3 144 962
- WO-A1-2014/199525
- WO-A2-2009/134929
- JP-A- 2009 084 258
- JP-A- 2009 084 258
- JP-A- 2011 001 271
- JP-A- 2011 001 271
- JP-A- 2013 538 803
- JP-A- 2015 509 725
- US-A1- 2015 343 399
- MASAMI NAGATA et al.: "Nano Bubble Suichu no Nano Bubble no Kaiseki = [Analysis of nanobubbles in nanobubble cage]", NanotechJapan Bulletin, vol. 8, no. 4, 2015, pages 1-6, XP009516480,
- HIDEHIRO YASUDA et al.: "Kuki Nano Bubble-sui no Kansatsu to Size Keisoku", [Test report Observation and size measurement of harmful air nano bubble water], 2014, page 1, XP009513298,

## Description

### Technical Field

The present invention relates to an administrable aqueous solution to a living body and a method for manufacturing the same, the administrable aqueous solution having a high effect of protecting the cells by minimizing damages and injuries to the cells under a hypoxic or anaerobic stimulation.

### Background Technology

As described in Non-Patent Literature 1, the features of micro-nano bubble typically include:
(a) bubble system is small,
(b) bubble rising speed is low,
(c) bubbles reduce frictional resistance,
(d) internal pressure of bubble is high,
(e) gas-liquid interface is large,
(f) amount of gas-dissolution is large,
(g) dissolution or shrinkage accompanies bubbles, and
(h) surface of bubble is negatively charged.

In particular, it is well known that liquids containing nanobubbles cannot be visually detected, resulting in being colorless and transparent, because nanobubbles are extremely small with a particle size of less than 1 *µ*m, and that the nanobubbles can remain in the liquid without floating on the top surface for a long period of time, because a buoyancy becomes extremely small compared to a viscous force as the particle size decreases. The nanobubble is expected to be applied to the medical area by taking advantage of these features.

For example, Patent Literature 1 discloses an infusion solution containing nanobubbles having an air bubble diameter of 1 to 1000 nm, preferably 50 to 500 nm. The invention disclosed in Patent Document 1 provides the infusion solution having a high oxygen content to compensate for the shortage of "dissolved oxygen" having an ability to supply oxygen to peripheral cells, because the ratio of "dissolved oxygen" is extremely low in "bound oxygen" bound to an erythrocyte and "dissolved oxygen" dissolved herein. This infusion solution is aimed to be used in "cerebral cryotherapy," "cerebral anemia," or "treatment for peripheral circulation insufficiency". When this infusion solution is applied to a selective coolant of the brain in rats, a high oxygen partial pressure can be achieved compared to that in infusion ones (Ringer's solutions) containing no oxygen nanobubbles. Patent Literature 1 discloses that the saline solution containing oxygen nanobubbles in Embodiment 1 can be manufactured by the method described in Patent Literature 2.

In addition, Patent Literature 3 has proposed infusion formulations containing NK (Natural killer) activation intensifier lymphocytes, NK activity enhanced mononuclear cells, or cell immunizing preparation, which were prepared by being cultured in the presence of NK activity enhancer containing nanobubble water as an active ingredient. Here, the nanobubble water containing air bubbles has a number mean diameter of 300 nm when determined by a dynamic light scattering method. The invention described in Patent Literature 3 was made by finding that the nanobubble water containing fine air bubbles with the number mean diameter of 300 nm or less when determined by the dynamic scattering method had an action of enhancing NK activity. In fact, the enhancement of the NK activity was confirmed using the oxygen nanobubbles having the volume mean particle diameter of 0.3734pm (373, 4nm) and a number mean diameter of 0.2995 pm (299, 5nm).

In addition, Patent Literature 4 discloses an aqueous fluid containing an ionic aqueous solution with an oxygen-containing nanostructure in which oxygen-containing nanobubbles with a mean diameter of less than about 100 nm were stably formed in an ionic aqueous stream and stabilized electrostatically. The aqueous fluid was used as the electrically modified aqueous fluid for preparing therapeutic medicines of cardiovascular diseases or their state. It is also described that a saline was included in the ionic aqueous solution.

On the other hand, the infusion solution or the physiological saline solution containing nanobubbles have been studied as one of drugs for the treatment or prevention of cancer as well as therapeutic purposes as described above. Lor example, Embodiment 1 of Patent Literature 5 proposes that ozone nanobubble water, and nanobubble water with a salt concentration of 0.3 mass%, which was mixed with oxygen nanobubble water and ozone nanobubble water, were applied to the treatment or prevention of cancer. It is described that the nanobubble water used at this time was manufactured by the method described in Patent Literature 2. Further, Patent Literature 6 discloses a cleaning method and cleaning device for cleaning with micro/nano-bubbles, Patent Literature 7 relates to a device for producing microbubble water by using a ultrasonic vibrator, Patent Literature 8 discloses a method for generating micro-nano bubbles, and Patent Literature 9 discloses a medicine containing nano-bubbles for the treatment or prevention of a cancer. Also Patent Literature 10 relates to an infusion (or fluid replacement) for efficiently and stably delivering oxygen to an organ such as brain in a low-temperature surgery, Patent Literature 11 discloses electrokinetically-altered fluids comprising an ionic aqueous solution of charge-stabilized oxygen-containing nanostructures, and Patent Literature 12 discloses a composition that contains protein and water containing ultra-fine air bubbles.

### Prior Art References

### Patent Literature

Patent Literature 1: Japanese Unexamined Patent Application Publication No. 2011-1271
Patent Literature 2: Japanese Unexamined Patent Application Publication No. 2005-246294
Patent Literature 3: Japanese Unexamined Patent Application Publication No. 2010-75180
Patent Literature 4: Japanese Translation of PCT International Application Publication No. JP-T-2013-538803
Patent Literature 5: Japanese Unexamined Patent Application Publication No. 2009-84258
Patent Literature 6: EP 3144962
Patent Literature 7: US 2015/343399
Patent Literature 8: EP 3009184
Patent Literature 9: JP 2009-084258
Patent Literature 10: JP 2011-001271
Patent Literature 11: WO 2009/134929
Patent Literature 12: EP 2772536

### Non-patent Literature

Non-Patent Document 1: Hideki Tsuge, "Fundamentals of Microbubble Nanobubbles," Bull. Soc. Sea Water Sci., Jpn., 2010, Vol. 64, p.4-10.

### Summary of the Invention

### Problems to be solved by the Invention

Aqueous solutions containing oxygen nanobubbles have been studied to be applied to administrable aqueous solutions to a living body such as physiological saline solutions, infusion solutions, or cell culture solutions, and have a certain degree of effect in improvement of oxygen supply and various treatments. However, the concrete applications have not progressed from the viewpoint of ensuring medical efficiency, safety, and security, since the effects are smaller than those expected, there is a fluctuation in the effects, and the effects are not stably persistent. In order to apply the aqueous solution containing oxygen nanobubbles to physiological saline solutions, infusion solutions or cell culture solutions, it is essential that its effects are clearly demonstrated and can be obtained stably and sustained.

In the administrable aqueous solution to a living body described above, the physiological saline solution is particularly useful because it is used as a solvent for infusions, or blood transfusions or injectable medicines containing various additives. Therefore, there is an extremely high need for the physiological saline solutions that have a function of supplying peripheral cells with sufficient oxygen, have less damage and injury to the cells under a hypoxic or anaerobic stimulation, and can enhance the effect of protecting the cells.

Although the infusion solution described in Patent Literature 1 above was defined the oxygen nanobubbles to have an air bubble diameter of 1 to 1000 nm, the air bubble diameter of the oxygen nanobubbles contained in the physiological saline solution actually used for produce the infusion solution was 50 to 500 nm. Patent Literature 1 above describes that a higher partial oxygen pressure was observed in the infusion solution containing oxygen nanobubbles, compared to that in an infusion solution (Ringer's solution) containing no oxygen nanobubbles. However, its effect is not quantitatively verified, and it is unknown whether it actually had an effect of protecting cells under the hypoxic or anaerobic stimulation. According to the study by the inventors of the present invention, it has been confirmed that the infusion containing oxygen nanobubbles had little effect of protecting cells in the presence of the hypoxic or anaerobic stimulation, and that there was little difference in superiority compared to the oxygen nanobubble-free saline solution, when the air bubble diameter of the oxygen nanobubble contained in the administrable aqueous solution to a living body such as the physiological saline solution was 50 nm or more. It has been found that the oxygen nanobubble water described in Patent Literature 2 above, which was manufactured by the same method as the invention described in Patent Literature 1 above, does not sufficiently produce an effect of protecting cells under the hypoxic or anaerobic stimulation.

The infusion formulation described in Patent Literature 3 above had oxygen air bubbles with a number average diameter of 300 nm or less when determined by a dynamic light scattering method, but the air bubble diameter of the oxygen nanobubbles actually studied in one of embodiments was 299.5 nm in the number mean diameter. Accordingly, it is unknown whether it has a sufficient effect of protecting cells under the hypoxic or anaerobic stimulation, as in Patent Literatures 1 and 2 above. In view of the results of the above studies conducted by the inventors of the present invention using physiological saline solutions having oxygen nanobubbles with an air bubble diameter of 50 nm or more, the invention described in Patent Literature 3 above is also difficult to obtain the effects of protecting cells under the hypoxic or anaerobic stimulation.

In addition, Patent Literature 4 above discloses the aqueous fluid in which oxygen-containing nanobubbles having a mean diameter of less than about 100 nm were stably formed in an ionic aqueous stream. However, the mean diameter of the oxygen-containing nanobubbles has not been actually measured, and was only estimated to be less than 100 nm, based on the measurement results of dissolved oxygen when passing through filters with a pore diameter of 0.22 and 0.1 micron. Furthermore, a density of the oxygen-containing nanobubbles contained in the aqueous fluid was not described in Patent Literature 4 above. It is unknown whether it was a sufficiently effective aqueous fluid to protect the cells under the hypoxic or anaerobic stimulation.

Furthermore, a density of the oxygen-containing nanobubbles contained in the aqueous fluid was not described in Patent Literature 4 above. It is unknown whether it was a sufficiently effective aqueous fluid to protect the cells under the hypoxic or anaerobic stimulation. This is because the nanobubble water used in the prior arts had the air bubble diameter of at least 50 nm or more. The resulting nanobubbles are likely to disappear rapidly depending on the operating environments such as the temperature and storage conditions, and not to function sufficiently in terms of absorption or permeability to cells and blood vessels in the body. In the invention of Patent Literature 5 above, the nanobubble waters used were made by the same method as described in Patent Literature 2, and the air bubble diameter of the nanobubble water was 50 nm or more.

The present invention has been made to solve the conventional problems described above, based on the verification that the effect of protecting cells can be sufficiently obtained under the hypoxic or anaerobic stimulation when the mean particle size and the density of oxygen nanobubbles contained in administrable aqueous solutions to a living body such as physiological saline solutions is respectively smaller and higher than those of the prior arts. The present invention is to provide an administrable aqueous solution to a living body and a method for manufacturing the same by not only reducing the mean particle size of oxygen nanobubbles contained therein, but also optimizing the density of oxygen nanobubbles in an increasing direction, by which an effect of protecting cells by reducing damages or injuries to the cells under the hypoxic or anaerobic stimulation and an effect of suppressing or preventing the proliferation and hypertrophy of cancer cells that are likely to occur in an anaerobic environment, can be increased and stably sustained.

### Means for Solving Problems

The present invention has been made by finding that the above-described problems can be solved by reducing the mean particle size to less than 50 nm and defining the density in a higher direction with respect to oxygen nanobubbles contained in administrable aqueous solutions such as physiological saline, and further by applying a formation method of the oxygen nanobubbles having such properties and characteristics to a method for manufacturing the administrable aqueous solution.

The scope of the invention is defined by the claims. Any references in the description to methods of treatment refer to the products of the present invention for use in a method for treatment. [0017]

Thus, the configuration of the present invention is as follows.
[1] The present invention provides an administrable aqueous solution to a living body,
   the administrable aqueous solution comprising an aqueous solution containing oxygen nanobubbles having a mean particle size of 30 nm or less and a density of 10¹⁶ bubbles or more per 1ml of the aqueous solution,
   wherein the mean particle size and the density of the oxygen nanobubbles are determined by a measurement with a cryo-transmission electron microscope using an ice-embedded method, characterized in that the administrable aqueous solution is for use in a method of inhibiting or preventing the proliferation and hypertrophy of cancers, wherein the method comprises the administration or oral ingestion of the administrable aqueous solution to the living body.
[2] The present invention provides the administrable aqueous solution to a living body according to item [1] above,
   the administrable aqueous solution comprising an aqueous solution containing oxygen nanobubbles having a mean particle size of 1 to 10 nm and a density of 10¹⁷ bubbles or more per 1ml of the aqueous solution,
   wherein the mean particle size and the density of the oxygen nanobubbles are determined by the measurement with the cryo-transmission electron microscope using the ice-embedded method.
[3] The present invention provides the administrable aqueous solution to a living body according to item [1] or item [2] above,
   wherein the administrable aqueous solution is a physiological saline solution comprising: the aqueous solution containing oxygen nanobubbles; and sodium chloride of 0.85 to 0.95 % by mass based on 100 parts by mass of the physiological saline solution.
[4] The present invention provides the biological administrable aqueous solution to a living body according to any of items [1] to [3] above,
   wherein the administrable aqueous solution is an infusion solution selected from the group consisting of hypotonic composite electrolyte solutions supplemented with 5 mass% of glucose solution, Ringer's solutions, and physiological saline solutions with a high-calorie fluid and heparin, which comprise the aqueous solution containing oxygen nanobubbles.
[5] The present invention provides the administrable aqueous solution to a living body according to any of items [1] to [4] above,
   wherein the administrable aqueous solution is a cell culture solution comprising the aqueous solution containing oxygen nanobubbles.
[6] The present invention provides the administrable aqueous solution to a living body according to items [1] to [4] above,
   wherein the administrable aqueous solution is used by an administration or an oral ingestion to the living body to inhibit or prevent the proliferation and hypertrophy of cancers.
[7] The present invention provides a method for manufacturing an administrable aqueous solution to a living body, the method comprising:
   producing jets of an aqueous solution containing dissolved-oxygen by injecting the aqueous solution from an outside of a cylinder via two or more small through-holes in the cylinder , at a pressure higher than the atmospheric pressure, the through-holes being arranged in a circumferential direction thereof with such a configuration that the respective openings of the two or more small through-holes are arranged facing each other on the same plane parallel to the radial cross section of the cylinder;
   creating a collision of the jets of the aqueous solution inside the cylinder so as that a water hammer of the jets concentrates at the center thereof; and
   generating oxygen nanobubbles by the mutual collision of the jets of the aqueous solution,
   wherein the oxygen nanobubbles have a mean particle size of 30 nm or less and a density of 10¹⁶ bubbles or more per 1ml of the aqueous solution,
   wherein the mean particle size and the density of the oxygen nanobubbles are determined by a measurement with a cryo-transmission electron microscope using an ice-embedded method.
[8] The present invention provides the method for manufacturing the administrable aqueous solution to a living body according to item [7] above,
   wherein the oxygen nanobubbles have a mean particle size of 1 to 10 nm and a density of 10¹⁷ bubbles or more per 1ml of the aqueous solution,
   wherein the mean particle size and the density of the oxygen nanobubbles are determined by the measurement with the cryo-transmission electron microscope using the ice-embedded method.
[9] The present invention provides the method for manufacturing the administrable aqueous solution to a living body according to item [7] or item [8] above, wherein the oxygen bubbles are generated by a bubble generation means, the bubble generation means comprising:
   a means for sucking each of oxygen and liquid;
   a means for pressurizing the oxygen and the liquid in a lump and transferring them;
   an oxygen-liquid mixing vessel for enriching the dissolved oxygen by mixing the transferred liquid, which includes the oxygen, with another new oxygen; and
   a means for generating nanobubbles using the dissolved liquid of the oxygen-liquid mixing state prepared in the oxygen-liquid mixing vessel,
   wherein the means has a bubble generating nozzle for generating nanobubbles, using a water hammering power caused by the mutual collision of the dissolved liquid with oxygen,
   wherein the bubble generating nozzle comprises: the cylinder having two or more small through-holes, arrayed in the circumferential direction thereof with such a configuration that the respective opening of each of such two or more small through-holes faces each other in the same plane; and a nanobubble discharge port provided on at least one end of the hollow cylinder,
   wherein the small through-holes are arranged so as that all of their extension lines passing through respective center of the cross-section of each of the small through-holes intersect each other in the inside of the hollow of the cylinder.
[10] The present invention provides the method for manufacturing the administrable aqueous solution to a living body according to any of items [7] to [9],
   wherein four to eight numbers of the small through-holes are equidistantly arranged in the circumferential direction of the cylinder, facing each on a same plane parallel to a radial cross-section parallel of the cylinder, and the pore diameter of the portion leading to the hollow of the cylinder is 0.1 to 0.5 mm.
[11] The present invention provides the method for manufacturing the administrable aqueous solution to a living body according to any of items [7] to [10] above,
wherein the administrable aqueous solution is a physiological saline solution prepared by adding sodium chloride to the aqueous solution containing oxygen nanobubbles at a content of 0.85 to 0.95 % by mass based on 100 parts by mass of the physiological saline solution.
[12] The present invention provides the method for manufacturing the administrable aqueous solution to a living body according to item [11] above,
   wherein the administrable aqueous solution is a physiological saline solution manufactured by using an aqueous solution containing 0.85 to 0.95 % by mass of sodium chloride based on 100 parts by mass of the aqueous solution, as the dissolved liquid of the oxygen-liquid mixing state.
[13] The present invention provides the method for manufacturing the administrable aqueous solution to a living body according to item [11] above, the method comprising:
   preparing the aqueous solution containing oxygen nanobubbles using an aqueous solution having no sodium chloride as the dissolved liquid of the oxygen-liquid mixing state; and
   manufacturing the physiological saline solution by adding sodium chloride to the aqueous solution containing oxygen nanobubbles at the content of the 0.85 to 0.95 % by mass based on 100 parts by mass of the physiological saline solution.
[14] The present invention provides the method for manufacturing the administrable aqueous solution to a living body according to any of items [7] to [13] above,
   wherein the administrable aqueous solution is an infusion solution manufactured by adding an additive to the administrable aqueous solution
   wherein the additive contains at least one selected from the group consisting of at least one element of potassium and calcium, 5% glucose solution, an amino acid, and heparin.
[15] The present invention provides the method for manufacturing the administrable aqueous solution to a living body according to any of items [7] to [14] above,
   wherein the administrable aqueous solution is manufactured as a cell culture solution for culturing cells.
[16] The present invention provides the method for manufacturing the administrable aqueous solution to a living body according to any of items [7] to [14] above,
   wherein the administrable aqueous solution is manufactured as an aqueous solution used by administration or oral ingesting to the living body to inhibit or prevent proliferation and hypertrophy of cancers.

### Advantageous Effects of the Invention

The administrable aqueous solution to a living body according to the present invention contains a large amount of oxygen nanobubbles having a smaller mean particle size than that in the prior art, which has the function of sufficiently supplying oxygen to peripheral cells, resulting in enhancement of the effect of protecting cells due to less damage or injury to cells under the hypoxic or anaerobic irritation. Furthermore, not only is the effect of stably protecting cells over the long period of time, but it can also greatly reduce variations of its effect. Furthermore, not only is the effect of protecting cells stable over the long period of time, but it can also greatly reduce variations of its effect.

Furthermore, since the administrable aqueous solution to a living body according to the present invention has the air bubble diameter of 30 nm or less, preferably 1 to 10 nm, which is smaller than that in the prior arts, the lifetime of the nanobubble is not affected by the operating environment such as temperature or storage condition, and the nanobubble water containing such the nanobubbles is excellent in its absorbability and permeability into cells, blood vessels, etc. in the living body. Therefore, the effect of inhibiting proliferation and hypertrophy of cancer cells that are likely to occur in an anaerobic environment, can be obtained stably and continuously.

In the method for manufacturing the administrable aqueous solution to a living body according to the present invention, the oxygen nanobubbles with the mean particle size of 30 nm or less can be also produced in large quantities and in a stable manner, compared to ones obtained using conventional nanobubble generators. Therefore, the administrable aqueous solution to a living body can be easily manufactured, which has the effect of protecting cells with little damages or injuries under the hypoxic or anaerobic stimulation, and the effect of inhibiting or preventing the proliferation and hypertrophy of cancer cells that is likely to occur in the anaerobic environment. And these effects can be obtained stably and continuously. The administrable aqueous solution to a living body having such the effects was hardly manufactured in the prior arts, but can only be obtained by the process of the present invention.

### Brief Description of Drawings

FIG. 1A and FIG. 1B are a front view and a perspective view showing an oxygen nanobubble generator for manufacturing an administrable aqueous solution to a living body according to the present invention, respectively
FIG. 2A and FIG. 2B illustrate an example of a nozzle shape for generating an oxygen nanobubble and a nozzle header for injecting a process liquid in the oxygen nanobubble generator shown in FIG. 1.
FIG. 3 illustrates one shape of the liquid collision nozzle 12 shown in FIG. 2.
FIG.4 illustrates photos of electronic microscope images of the amorphous ices of water and a particle size distribution of the nanobubbles for air nanobubble water in Reference Embodiment 1.
FIG. 5 illustrates a photograph of an electronic microscope image of the amorphous ice of water for oxygen nanobubble water in Embodiment 1.
FIG. 6 illustrates a number distribution of bubble particle sizes of the oxygen nanobubble water by a measurement using a dynamic light scattering method in Embodiment 1.
FIG. 7 illustrates photos showing morphological changes in cells before and after left in an anaerobic environment in Embodiment 1 and Comparative Example 1.
FIG. 8 illustrates the measurement results of the amounts of enzyme (LDH) leaking out during cytotoxicity before and after left under the anaerobic atmosphere in Embodiment 1 and Comparative Example 1.
FIG. 9 illustrates the measurement results of cell growth (cell viability) before and after left the anaerobic environment in Embodiment 1 and Comparative Example 1.
FIG. 10 illustrates phots showing the morphological changes in cells taken after left in the anaerobic environment in Embodiment 1 and Comparative Example 2.
Fig. 11 illustrates the cell viability measured after left under the anaerobic atmosphere in Embodiment 1 and Comparative Example 2.
FIG. 12 illustrates a commercially available hypoxic culture kit used for the culture of cancer cells in Embodiment 3 and Comparative Example 3.
FIG. 13A and FIG. 13B illustrate the induction results of HIF-1a and HSC70 proteins after left in a hypoxic atmosphere with O₂ concentration of about 1% for 6 and 24 hours in Embodiment 3 and Comparative Example 3.
FIG. 14 illustrates the procedure for converting areas of the black portions shown in Fig. 13A in numerical form by an image processing.

### Modes for Implementing the Invention

As described in Non-Patent Literature 1, various methods for generating gas micro-nanobubbles have been proposed, such as a swirling liquid flow type, a static mixer type, a venturi type, a pressurized solubility type, and a pore type. In addition, as described in the Patent Literatures 1 and 4 above, a method for generating ozone nanobubbles having a finer air bubble diameter (the air bubble diameter of 50 to 500 nm) by giving physical stimulation to oxygen microbubbles contained in an aqueous solution has also been proposed. However, the air bubble diameter obtained by these conventional nanobubble generating methods is limited in about 50 nm, even with the smallest one. Furthermore, since gaseous nanobubbles with 100 nm or less are very fine particles with a smaller diameter than the wavelengths of visible light and ultraviolet light, measurement techniques capable of precisely measuring particle size have not been established. What is commonly referred to as the aqueous solution containing gas nanobubbles was difficult to clearly demonstrate the presence of nanobubbles with the particle size of several tens of nanometers or less.

The inventors of the present invention have verified the effectiveness for protecting cells under the hypoxic or anaerobic stimulation using physiological saline solutions containing oxygen nanobubbles with 50 nm as the smallest air bubble diameter, which were obtained by a conventional micro-nanobubble generation method. Verification was done by quantitatively and qualitatively measuring changes in cell morphology and the extent of cell damage under the anaerobic (hypoxic) stimulation in a state of mixing a physiological saline solution containing oxygen nanobubbles with a cell culture solution at a concentration of 50 mass% to determine the extent to which the cell protection can actually be achieved. The results showed that the degree of cell damage was almost the same as that of a normal physiological saline solution without oxygen nanobubbles, and that the physiological saline solution containing oxygen nanobubbles with the air bubble diameter of 50 nm or more caused large cell injuries, resulting in increased cell damages. Therefore, it has been clarified that it is not sufficient to just define that the bubble size of ozone nanobubbles contained in the physiological saline solutions is generally less than 1000 nm, less than 500 nm, less than 300 nm, or less than 100 nm to obtain the effect of protecting cells.

According to the above verification results on the protecting effect of cells under the hypoxic or anaerobic stimulation , the present invention has been made by studying a method for manufacturing an physiological saline solution containing oxygen nanobubbles with such properties and characteristics by trial and error, and finding a method that is feasible, based on the idea that the protecting effect of cells under the hypoxic or anaerobic stimulation may be obtained sufficiently by increasing the density of the ozone nanobubbles contained in the physiological saline solution, as well as by further reducing the mean particle size of the oxygen nanobubbles therein, compared to the conventional methods. By using the physiological saline solution actually produced by the method according to the present invention as a cell culture solution, the protecting effect of cells under the hypoxic or anaerobic irritation was confirmed. The verification results will be described in detail in the embodiments described below.

The size of the oxygen nanobubbles in the physiological saline solution according to the present invention can be defined by the mean particle size. The smaller the mean particle size, the higher the amount of bubbles contained at the nano level, and the smaller the amount of bubbles with larger particle sizes. The size of the micro-nano bubbles is also influenced by particle size distributions (the standard deviation of particle size), but the effect is small. The nanobubbles contained in the physiological saline are required for having a mean particle size on the order of less than 50 nm and as small as possible.

In the present invention, the mean particle size of oxygen nanobubbles are not more than 30 nm and preferably not less than 1 nm and not more than 10 nm, when measured with a cryo-transmission electron microscope by a ice-embedding method. When the mean particle size of the oxygen nanobubbles is 30 nm or less, the high effect of protecting cells under the hypoxic or anaerobic stimulation can be obtained stably and continuously due to less damages of injuries of cells. In addition, a significant effect can be achieved if the mean particle size is 10 nm or less. On the other hand, the effect of protecting the cells tends to be saturated even when the mean particle size of the oxygen nanobubble is less than 1 nm, and it is preferable to define the average particle size of 1 nm or more from the viewpoint of economic efficiency and easy maintenance in light of the high technical hurdles involved in assembling an oxygen nanobubble generator.

In the present invention, it is necessary to define not only the mean particle size of the oxygen nanobubbles, but also the number contained in 1 ml of the administrable aqueous solution to a living body such as a physiological saline solution, i.e., the density of the oxygen nanobubble. This is because it is necessary to increase the total amount of oxygen contained in 1 ml of the administrable aqueous solution to a living body, such as the physiological saline solution, in order to sufficiently exert the function of protecting cells under the hypoxic or anaerobic stimulation. On the other hand, the storage stability and the particle size maintenance of the bubble need to be improved by making the mean particle size of the oxygen nanobubbles very small, so as to be 30 nm or less.

The density of oxygen nanobubbles contained in the administrable aqueous solution to a living body such as the physiological saline solution, used in the present invention, need to be 10¹⁶ bubbles or more, preferably 10¹⁷ bubbles or more per 1 ml of the administrable aqueous solution, as determined by the measurement with the cryo-transmission electron microscope using the ice-embedding method. In the first place, the oxygen nanobubbles in the present invention are very small in the mean particle size. If the density of oxygen nanobubbles is less than 10¹⁶, the oxygen concentration per unit volume of the administrable is low, and the effect of protecting cells under the hypoxic or anaerobic stimulation is insufficient. The effect of protecting cells under the hypoxic or anaerobic stimulation increases with increasing the oxygen concentration. In addition, when the mean particle size of the oxygen nanobubbles is 1 to 10 nm, the density of the oxygen nanobubble is preferably 10¹⁷ bubbles/ml or more in order to ensure the adequate oxygen concentration contained in the administrable aqueous solution to a living body.

Various methods for measurement the particle size of micro-nanobubbles have been known. Among these, the measurement method of nanobubbles by optical observations is difficult. Therefore, the measurement methods such as a light scattering method using the Mie scattering light, a laser diffraction/scattering method, a nanoparticle tracking error analysis method for observing the Brownian motion of bubble particles in a solution, a resonant mass measuring method using beams of pore resistances (a call counter method), a dynamic light scattering method, and MEMS (Micro Electrical-Mechanical Systems) have been proposed. In addition to these methods, a method for determining the particle size of nanobubbles by a Zeta potential measurement and a method for confirming the presence of nanobubbles by electronic sponge resonance (ESR) using a spin trap have been proposed.

The inventors of the present invention have proposed a method of measuring micro-nanobubble with a cryo-transmission electron microscope by the ice-embedding method (see Japanese Examined Patent Application Publication No. 2014-230407). In this method, the superfine bubbles contained in the liquid are solidified inside a liquid in an amorphous solid state and then the liquid in the amorphous solid state can be viewed with the transmission electron microscope and analyzed by directly observing and analyzing the superfine bubbles contained in the liquid as observation images. Therefore, ultrafine-fine bubbles with the particle size of less than 10 nm can be accurately measured. In addition to the mean particle size of the oxygen nanobubbles, this method can also determine their particle size distribution and density. Therefore, the mean particle size and the density of the ozone nanobubbles defined in the present invention are determined by this method.

Cryo-transmission electron microscope measurements by the ice-embedding method are performed by setting 1 to 10⁵ electrons per Å² for the number of electrons used in the observation with a transmission electron microscope operated under the energy of 10 to 300 kilo-electron bolts (keV), using a micro grid or a liquid held in a micromesh as a sample.

The bubble particle size of oxygen nanobubbles water in the present invention can be measured by, for example, a dynamic light scattering method (a photon correlation method), in addition to the cryo-transmission electron microscope measurements by the ice-embedding method. For example, specific data processing can be performed using a particle size/molecular weight measuring equipment (Designation: ELSZ-2000 S) manufactured by Otsuka Electronics or a Zeta potential/particle size/molecular weight measurement system (model number: ELSZ-2000 ZS) to measure the bubble size of 10 nm or less. Here, the specific data processing can be performed by, for example, a method of measuring the particle size by extracting only the particles that are stably present when increasing the accumulated number of measurements and deleting only the data that is uncertain and randomly reflected at the time of measurement.

As the result of the study by the inventors of the present invention, it was confirmed that the measurement results of the particle size by the dynamic light scattering method are similar to those with the cryo-transmission electron microscope by the ice embedding method. The comparison between the two measurement methods will be described with concrete data shown in Embodiment 1 below. However, in the dynamic light scattering method, it is extremely difficult to clearly distinguish whether the measured particles are filled or unfilled. The high-precision measurement of oxygen nanobubble density is also difficult due to technical constraints. Compared to the dynamic light scattering method, the cryo-transmission electron microscope measurement, as in the present invention, allows not only to clearly distinguish the difference between a filled particle and an unfilled particle by the measurement with the electron microscope, but also to measure the density of oxygen nanobubbles with a high precision. Accordingly, the present invention employs a method of measuring the oxygen nanobubble diameter using the cryo-transmission electron microscope by the ice embedding method.

The administrable aqueous solution to a living body according to the present invention can be used as a physiological saline by containing basically sodium chloride of 0.9 mass% so as that the osmotic pressure thereof is compatible with that of blood or body fluids, as in normal physiological saline solutions. The sodium chloride content in the physiological saline solution according to the present invention is acceptable even if there are variations to some extent. However, its use is greatly limited when the sodium chloride content is extremely separated from 0.9 mass %. Thus, it is preferable to contain sodium chloride in the range of 0.85 to 0.95 mass% by weight based on 100% by weight of the physiological saline solution, when the administrable aqueous solution to a living body according to the present invention is used as a physiological saline solution.

The physiological saline solution according to the present invention may contain a small amount of hydrogen nanobubbles or other electrolyte solutions when adjusting its redox potential to be matched with that in the organ or in vivo reaction of the human body. In order to adjust the pH of the physiological saline solution, a pH adjusting agent composed of electrolytes or the like may also be added.

The administrable aqueous solution according to the present invention can also be used as an infusion solution containing various additives, such as the following. For example, the infusion solution comprises a saline solution obtained by adding a hypotonic complex electrolyte solution with 5% glucose solution added in a predetermined amount, a Ringer's solution with potassium or calcium, a high calorie solution with glucose or amino acids, or heparin, to the physiological saline according to the present invention. In the preparation of the infusion solution according to the present invention, the physiological saline solution obtained from the present invention is usually used as a solvent, but is not necessarily limited to a physiological saline solution containing sodium chloride in the range of 0.85 to 0.95 mass %. When the infusion solution according to the present invention is administered to a living body, an aqueous solution containing no sodium chloride content or an increased content of sodium chloride may be used as the infusion solution containing the above-mentioned additives, if the osmotic pressure of the infusion solution does not necessarily need to be closely matched to that of the blood or body fluids.

The physiological saline solution or the infusion solution of the present invention can be used as an aqueous solution for culturing various cells, such as vascular smoothness muscle cells. For example, these solutions can be highly effective when attempting to reduce cell damages and injuries in hypoglycemic and hypoxic states during or after exercise and to enhance the protection against the cytotoxicity due to myocardial infarction, cerebral infarction, and other circulating disorders.

The administrable aqueous solution to a living body according to the present invention can also be used as an aqueous solution administrated or orally ingested to a living body to inhibit or prevent the proliferation and hyperplasia of cancer that occurs in an anaerobic atmosphere at a low oxygen concentration. It is known that cancer cells tend to grow under the anaerobic atmosphere. Therefore, it is believed that the proliferation and hypertrophy of cancer cells may be suppressed by forming the surroundings of cancer cells aerobic with high oxygen concentration. For this reason, for example, as disclosed in Patent Literature 5 above, it has been proposed that nanobubble water having an air bubble diameter of 50 nm to 500 nm may be used for the treatment of cancer. However, since the pore size on the surfaces of normal cells and blood vessels in vivo is 30 nm or less, specifically several nm to several tens of nm, the absorption or permeability of the oxygen nanobubbles to the cells or blood vessels in vivo is not necessarily adequate when the air bubble diameter present in the nanobubble water containing oxygen and ozone nanobubbles is 50 nm or more. Further, as the size of the nanobubbles becomes larger, the expansion of the air bubbles is accelerated, and the bubble disappearance is promoted, since the organism is normally maintained at a slightly elevated temperature of 35-37 °C. This resulted in the insufficient suppression or prevention effect against the proliferation and hypertrophy of cancer cells. Even if the effect had been effective, it was not stable.

Compared to the prior art, the oxygen nanobubbles contained in the administrable aqueous solution to the living body according to the present invention are air bubbles having the mean particle diameter of 30 nm or less, preferably from 1 to 10 nm, and also has the very high density in the bubbles, so that they are highly absorbable or permeable to in vivo cells and blood vessels and have a relatively long lifetime of the nanobubbles existing at the air bubble diameter having the same range. As the result, compared with conventional oxygen nanobubble water, even the aqueous solution containing only oxygen nanobubbles according to the present invention can maintain the surrounding environment of cancer cells in an aerobic state with a high oxygen concentration for a long period of time, thereby increasing the effect of suppressing or preventing the proliferation and hypertrophy of cancer cells. In addition, the effect can be sustained over a relatively long period of time. Thus, the administrable aqueous solution a living body according to the present invention has a function as a drug for suppressing or preventing the proliferation and hypertrophy of cancer, and is a suitable aqueous solution in the medical area such as the treatment of cancer.

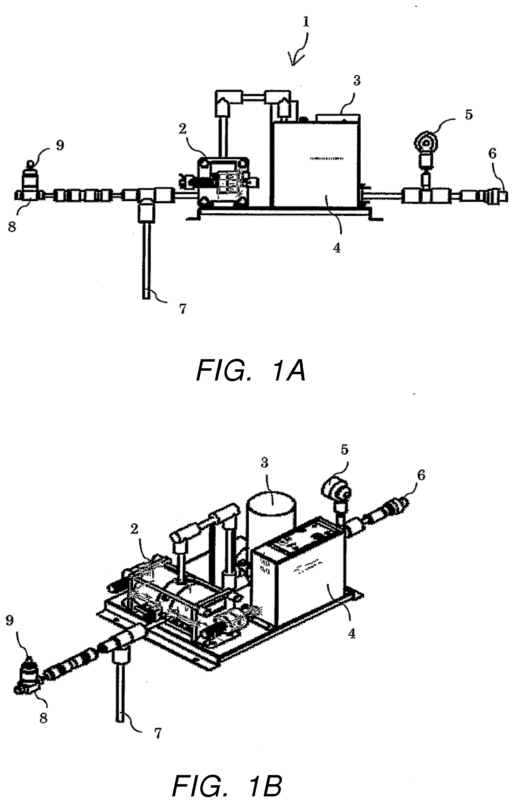

Next, an oxygen nanobubble generator for manufacturing the physiological saline solution according to the present invention will be described with reference to the drawings. FIG. 1A and FIG. 1B are a front view and a perspective view of the ozone nanobubble generator, respectively.

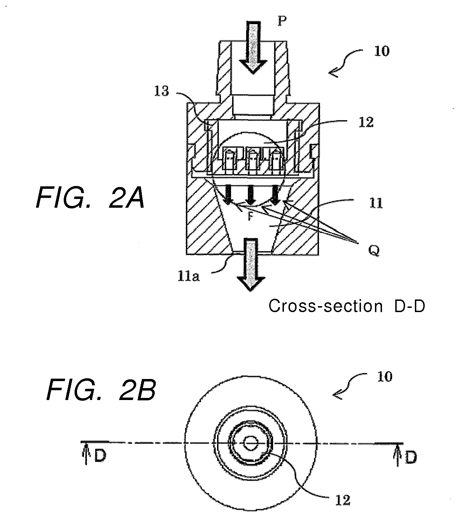

FIG. 1A and FIG. 1B illustrate an example of an oxygen nanobubble generator used in the present invention, and the basic configuration is the same as that described in Japanese Examined Patent Application Publication No. 555892. In the oxygen nanobubble generator 1 illustrated in FIG. 1A and FIG. 1B, 2 is a bellows cylinder pump, 3 is a gas-liquid mixing tank, 4 is a pump controller, 5 is a pressure sensor, 6 is a nozzle attachment for nanobubble generation, 7 is a liquid suction tube, 8 is a gas suction port, and 9 is a gas suction regulating valve.

These are arranged as shown in the perspective view shown in FIG. 1B. The volume of gas (oxygen) is adjusted with the bellows cylinder pump 2 made of a fluorinated resin, using the liquid suction tube 7 and the gas suction adjustment valve 9. Then, oxygen is dissolved inside a pressurized liquid by sucking a liquid in a mixing state of liquid and oxygen inside the pump, agitating them and dissolving the gas inside the bellows. In the present invention, the bellows cylinder pump 2 may be metal-free, in which at least one type of plastic other than fluorinated resin may be used, such as general purpose plastics of such as polyethylene, polypropylene and polyethylene terephthalate, engineering plastics of such as polyacetals, polyamides, polycarbonates and degenerative polyphenylene ethers, and super engineering plastics such as polyether sulfones, polyphenylene sulfides, polyether ether ketones and liquid crystal polymers. In this case, a highly reliable clean oxygen nanobubble generator can be achieved by using the above-described various plastics, including fluorinated resin, not only in the pump but also in the liquid section. In this case, a highly reliable clean oxygen nanobubble generator can be achieved by using the above-described various plastics, including fluorinated resin not only in the pump but also in the liquid section. In the present invention, metals or ceramics as well as the above-described plastics may be also used, when cleaning or sterilization by strict metal-free conversion is not required.

Then, the gas and the liquid are stirred by the pump 2 and pumped to the gas-liquid mixing tank 3. As the pump 2, a compressed air-activated bellows cylinder pump is used, or an electrically operated pump may be used. The gas and liquid in the gas-liquid mixing tank 3 are subject to pressure from the pump 2, which facilitates the dissolution of the gas. That is, the pressure for pumping the gas and the liquid from the pump 2 is checked with the pressure sensor of 5. This method is performed for a preparing to increase an amount of dissolved gas to increase a generating amount of nanobubbles. Although it is practical to use the bellows cylinder pump as the pump 2 in the oxygen nanobubble generation system of the present invention, it is possible to apply reciprocating pumps, such as piston pumps, plunger pumps, or diaphragms, as well as rotating pumps, such as gear pumps, eccentricity pumps or screw pumps, cascade pumps, vane pumps, and the like, which are hitherto well known as feed pumps.

The liquid pumped into the gas-liquid mixing tank 3 is mixed with oxygen. Oxygen dissolved inside the liquid is fed to the nozzle attachment 6 for generating nanobubbles. The nozzle attachment 6 for generating nanobubbles corresponds to a part connected to a nozzle for generating a large amount of oxygen nanobubbles having the mean diameter of 30 nm or less, preferably 1 nm to 10 nm.

At this time, the pressure sensor 5 monitors the dissolved conditions of the gas in the liquid by observing the change of the liquid pressure between the nozzle 6 and the gas-liquid mixing tank 3. In this way, the constant pressure condition required for the generation nozzle to generate stable nanobubbles is controlled.

In the present invention, the bubble generating steps performed by using the oxygen nanobubbles generator shown in FIG. 1A and FIG. 1B are as follows. A step for sucking each gas and liquid is performed using the liquid suction tube 7, the gas suction port 8, and the gas suction adjustment bubble 9. The pressure is adjusted by the pressure sensor 5. Next, a step of pressurizing the liquid containing the gas including oxygen using the bellows cylinder pump 2 is the gas-liquid pressurization step. Subsequently, in order to mix the liquid containing the pressurized gas with new oxygen, a step performed using the pump controller 4 and the gas-liquid mixing vessel 3 is the dissolved gas enrichment step. After that, the generation nozzle used in the present invention as described below is connected to the nozzle attachment for generating oxygen nanobubbles 6, and then oxygen nanobubbles are generated. This step is referred to as the dissolved gas miniaturization step, wherein the oxygen nanobubbles can be generated by injecting from the outside of a cylinder having two or more small through-holes at a pressure higher than the atmospheric pressure via the small through-holes and by a mutual collision of jets at one point in the inside of the cylinder.

In the oxygen nanobubble generator used in methods according to the the present invention, the liquid from which the air is removed as much as possible may be used, wherein the liquid is obtained by treating a usual liquid containing the air under vacuum. The degassed liquid is used as the liquid containing oxygen nanobubbles by mixing with the oxygen aspirated from the gas inlet 8 and/or with the new oxygen in the dissolved gas enrichment step. And then the oxygen nanobubbles are generated by the bubble generation nozzles used in the present invention. This method is preferably employed in the manufacturing method according to the present invention, because it provides an effect of increasing the oxygen concentration in the liquid during the subsequent oxygen mixing and dissolution steps by degassing the liquid prior to mixing the oxygen.

FIG. 2A and FIG. 2B illustrate an example of a nozzle shape that generates oxygen nanobubbles and a nozzle header that injects the processing solution in the cleaning apparatus of FIG. 1, respectively. FIG. 2A and FIG. 2B are cross-sectional and top views of the nozzle header 10, respectively. FIG. 2A shows the D-D cross-section of FIG. 2B.

As shown in FIG. 2A and FIG. 2B, the nozzle header 10 is composed of the injection nozzle 11 for injecting a processing liquid, and the liquid collision nozzle 12 and the table 13 for discharging oxygen nanobubbles. One or more of the liquid collision nozzles 12 are mounted on the table 13. Here, the liquid collision nozzle 12 is an example of a nozzle shape for generating oxygen nanobubbles.

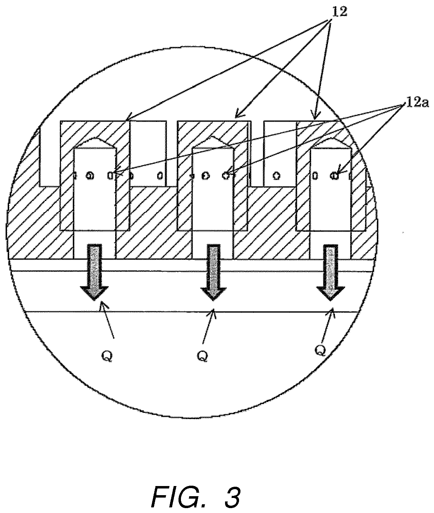

FIG. 3 is an enlarged view of a portion in which the liquid collision nozzle 12 of the nozzle header 10 is arranged as shown in FIGS. 2A and 2B. As shown in FIG. 3, in one shape of liquid collision nozzles 12, a small hole in 12a is opened toward the center of 12. Through this small hole 12a, jets of liquid entering under a high pressure are mutually collided at the center of the liquid collision nozzle 12 to generate nanobubbles and then ejected in the direction indicated by the arrow Q. Experiments have shown that controlling the speed V of the solution increases the amount of nanobubbles generated and the lifetime of the bubbles. As an indication of velocity V, the stable nanobubble generation nozzle is achieved at speeds exceeding 25 m/sec.

When preparing the aqueous solution containing oxygen nanobubbles, which has, for example, a desired additive such as sodium chloride, the aqueous solutions (Q) ejected from the liquid collision nozzle 12 in FIGS. 2 and 3 are adjusted in accordance with the following two. The first method is to use an aqueous solution containing a desired additive such as sodium chloride in a predetermined mixing amount, as the solution to be aspirated by the liquid suction tube 7 shown in FIGS. 1A and 1B. The aqueous solution containing sodium chloride obtained by dissolving oxygen to make the gas-liquid mixture is injected from the fuel injection nozzle 11 as it is. The aqueous solution containing oxygen nanobubbles together with sodium chloride is manufactured in this manner. In the second method, the aqueous solution containing oxygen nanobubbles is manufactured using an aqueous solution that does not contain additives such as sodium chloride (including pure water) as the solution to be aspirated by the liquid suction tube 7 shown in FIGS. 1A and 1B, and then is used as an aqueous solution containing additives by combining the additives such as sodium chloride at a predetermined content based on 100 parts by mass of the aqueous solution of.

In addition, when the aqueous solution (Q) is prepared as an infusion solution, an aqueous solution containing at least an additive selected from the group of at least one element of potassium and calcium, 5% glucose solution, amino acids, and heparin, can be used as the solution to be aspirated by the liquid aspiration tube 7 shown in Fig. 1. The gas-liquid mixture obtained by injecting from the injection nozzle 11 can be used as the infusion solution after the oxygen is dissolved in the aqueous solution. At this time, sodium chloride may be contained at the same time if necessary. In addition to this method, an aqueous solution without the additive is used as the solution to be aspirated by the liquid suction tube 7 shown in FIG. 1. After an aqueous solution containing oxygen nanobubbles is manufactured, this aqueous solution containing nanobubbles with an additive added at a predetermined content is used as an infusion solution. At this time, not only pure water but also a physiological saline solution containing sodium chloride may be used as the aqueous solution without the additive.

A method for generating oxygen nanobubbles using water flow injected from the liquid fuel injection nozzle 11 will be explained below. Since the pressure is rapidly released from the discharge pressure (higher than the atmospheric pressure) of the high pressure pump composed of the bellows cylinder pump 2, jets of the oxygen-dissolved liquid injected from the high speed jet liquid injection nozzle 11 collide with each other, forming the liquid in a state containing a large amount of oxygen nanobubbles by breaking the dissolved liquid with gas, using a bursting force attributable to the water hammering force of the jets. Conventional pressure releasing methods may reduce the amount of ozone nanobubbles generated. However, the equipment according to the present invention can generate a large amount of oxygen nanobubbles.

The oxygen nanobubbles contained in the physiological saline solution of the present invention may be generated at an amount equal to or higher than those of the conventional solution when the dissolved solution in the gas-liquid mixing state is injected at atmospheric pressure (about 0.1MPa) or higher, for example, by using the liquid impingement nozzle 12 having the structures shown in FIGS. 2 and 3. In addition, by setting the pressures to 0.2MPa or more, the aqueous solution containing a sufficient amount of ozone nanobubbles can be prepared. Thus, a suitable pump for eliminating the influences of metal contamination, such as the compressed air-driven or electrically operated bellows cylinder pump 2 made of fluoropolymer, can be used, since the lower limit of the injection pressure of the dissolved solution can be reduced to 0.2MPa, which is still lower than that in the prior arts. The upper limit of the injection pressure of the dissolved liquid with oxygen is not specified, but it is preferable to set it to 1.0MPa or less when the load of the oxygen nanobubble generation equipment 1 by the increase of the injection pressure is to be reduced.

The nozzles of the oxygen nanobubble generator used in manufacturing the physiological saline solution containing oxygen nanobubbles according to the present invention are designed so as that the jets of the dissolved solution can be injected even at a pressure higher than atmospheric pressure, preferably 0.2MPa or more, which is still lower than that in the prior arts. Let F be the water hammering force obtained by injection and collision of the jets of the gas dissolved solution. For the water hammering force F, the relationship of F = ρSV² is established when the density of the solution is ρ, the size of the small hole is S, and the speed of the solution is V. An optimized design that takes account of the relationship between the through-hole size S and the speed V is necessary for optimizing F.

In the oxygen nanobubble generator used in methods according to the the present invention, the diameter of the through-holes S, which are formed in the liquid collision nozzle shown in FIGS. 2 and 3, that is., the diameter of the small through-holes 12a is preferably 0.1 to 0.5 mm, and more preferably 0.2 to 0.4 mm. Here, when the diameter of the hole 12a of the liquid collision nozzle 12 is less than 0.1 mm, the generation amount of fine ozone bubbles with a small particle diameter tends to increase, but the generation amount of the bubbles with a particle size of 1 nm or more drastically decreases, resulting in a decrease in the generation amount of oxygen nanobubbles and a remarkable decrease in the density of the oxygen nanobubbles. In addition, when the diameter of the hole 12a of the liquid collision nozzle 12 exceeds 0.5 mm, the total generation amount of the bubbles having the particle size of 1 nm or more increases. On the other hand, the generation amount of the small particle size bubbles having the 10 nm or less decreases sharply. Therefore, the stability of the oxygen nanobubbles is drastically decreased with increasing the mean particle size, and the present invention cannot have a sufficiently effective effect. Accordingly, it is more preferred that the through-hole diameter of the liquid collision nozzle 12 in the present invention is in the range of 0.1 to 0.5 mm in order to reduce the mean particle size of the oxygen nanobubbles to 30 nm or less and to increase the density of the oxygen nanobubbles to 10¹⁶ bubbles or more per 1 milliliter of the aqueous solution. Furthermore, by providing a through-hole diameter of the liquid collision nozzle 12 in the range of 0.2 to 0.4 mm, the mean particle size and the density of oxygen nanobubbles can be 1 to 10 nm and 10¹⁷ bubbles or more per 1 ml of the aqueous solution, respectively.

The same effect can be achieved by injecting the dissolved liquid from four sides toward the center of the nozzle cylinder and concentrating water on the center thereof, and thereby increasing the speed, resulting in the generation of a large amount of oxygen nanobubbles with the smaller mean particle size. For this reason, in the case of water shooting from four sides, if the water injection speed is the same, a larger effect can be obtained depending on the number of holes in the through holes. For example, when four through-holes are equipped, the force to be collected in the center becomes F=4ρSV² by four times of F=ρSV² because each F of the through-holes is concentrated in the center. In that case, twice the water hammer power can be obtained as compared with that in the case of two through-holes. When the number of small holes in the nozzle is increased in order to concentrate the water hammering power created by the collision of the liquid jets in the center, the collision energy of the liquid is increased due to the high flow rate. Regarding the generation amount of oxygen nanobubbles, a large amount of oxygen nanobubbles with a smaller mean particle size can be generated, as the collision energy of the liquid increases.

In the present invention, the through-hole diameter of the liquid collision nozzle 12 is defined as 0.1 to 0.5 mm, thereby increasing the speed V of the dissolved solution with oxygen. By the increase of the speed V, the effect of reducing the mean particle size of the generated oxygen nanobubbles can be obtained. In addition, since the density of oxygen nanobubbles needs to be increased at the same time, the through-holes of the liquid collision nozzle 12 are preferably provided in the number of 4 or more and 8 or less at equal intervals in the circumferential direction of the cylinder of the liquid collision nozzle 12. If the number of through-holes in the liquid collision nozzle 12 is 3 or less, the density of oxygen nanobubbles is remarkably reduced. When the number of through-holes is 9 or more, not only the effect of increasing the density of the oxygen nanobubble is saturated, but also it is very difficult to manufacture the liquid collision nozzle 12, because a high accuracy is required for positioning of the through-holes in the liquid collision nozzle 12.

In the oxygen nanobubble generator used in methods according to the present invention, the shape of the liquid collision nozzle 12 is provided with four to eight small through-holes 12a, arranged at equal intervals in circumferential direction thereof. In addition, for example, the through-holes may be drilled in parallel with two or more rows in the longitudinal direction of the liquid collision nozzle 12 to create the water hammer of the liquid at two or more locations. This allows a large amount of nanobubbles to be generated, which is an effective method for miniaturization and efficiency improvement of the nozzle. Further, since the strength of the water hammer can be increased by injecting the liquid from four or more small through-holes at the same time, a large amount of oxygen nanobubbles with a mean particle size of 30 nm or less can be generated without increasing the speed V of the liquid. This eliminates pumps for discharging the liquid at high pressures and thereby reduces the burden, enabling energy-efficient nozzles to be developed by the highly useful technical in the industrial field.

### Embodiments

The following concretely describes embodiments of the present invention. However, these described embodiments in no way limit the scope of the present invention.

### <Reference Example 1>

Air nanobubble water was produced according to the method disclosed in Japanese Examined Patent Application Publication No. 555892 using the nanobubble water producing device Model ΣPM-5 (a bellows pump type, a product of Sigma Technology Co., Ltd.), and used as a measurement specimen after dilution with pure water by a factor of 100. The liquid collision nozzle 12 was used, as shown in FIGS. 2A and 2B. The through-holes formed in the liquid collision nozzle 12 are provided at six equal intervals in circumferential direction of the cylinder comprising the liquid collision nozzle 12 so as to face on the same plane parallel to the radial cross section of the cylinder, and the hole size of the portion leading to the cavity inside the cylinder is 0.3 mm. Pure water was also used as a reference sample before generating nanobubbles. Pure water prior to the nanobubble generation step corresponds to water that does not contain nanobubbles.

A sample of nanobubbles embedded in the amorphous ice of water was prepared by rapid freezing of the air nanobubble water immediately after the production thereof using the sample rapid freezing equipment of Vitrobot Mark IV (a product of FEI Co., Ltd.), and the sample was used as a sample for observation. The sample thickness was 200 nm. The nanobubbles embedded in amorphous ice were observed directly at a sample temperature of about 80K using a cryo-transmission electron microscope of Titan Krios with the electron energy of 300 keV (a product of FEI Co., Ltd.). The electronic beam used for observation was about 20 electrons/Å² according to the Low dose technical, and there was little increase in the sample temperature during imaging.

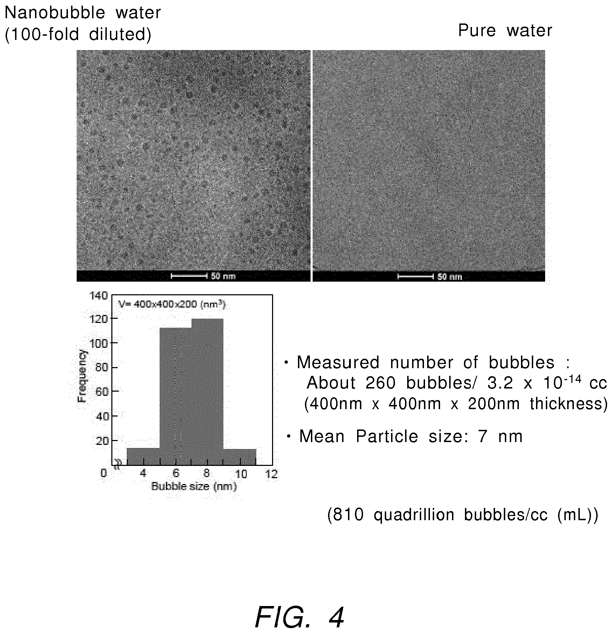

FIG. 4 illustrates photos of the electronic microscope images of the amorphous ices frozen from the pure water containing air nanobubbles and the amorphous ice frozen from pure water (water without nanobubbles), respectively. For the air nanobubble water, the size distribution of the bubbles (a histogram to show the particle size variance) is shown below the electronic microscope images.

The photograph of the electronic microscope image shown on the left side of FIG. 4 shows the image of air nanobubbles that were immediately observed after being produced by ΣPM-5. The circular contrasts observed in the photograph of FIG. 4 are nanobubbles. As the result of image processing, the average particle size is 7 nm. The volume of the amorphous ice used for the measurement of the histogram was 3.2 × 10⁻¹⁴ cc (400 nm×400 nm×200 nm thickness), in which approximately 260 bubbles are contained. Since the 100-fold-diluted nanobubble water was observed, the concentration of the air nanobubbles in the nanobubble water was evaluated to be 8.1 × 10¹⁷ bubbles/cc (ml) (810 quadrillion bubbles/cc (ml)). On the other hand, the photograph of the electronic microscope image shown on the right side of FIG. 4 shows no contrast change. From FIG. 4 it can be confirmed that the photograph means the amorphous ice of the pure water containing no bubbles. Thus, the measurement method and apparatus according to the present invention allow not only the presence of nanobubbles in water to be directly identified as the images, but also the information on the particle size, number, particle size distribution and the shape of the nanobubbles to be obtained.

### <Embodiment 1>

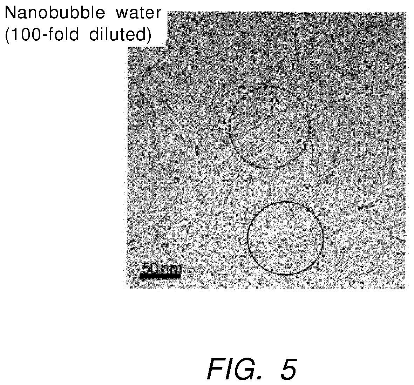

An aqueous solution containing oxygen nanobubbles was prepared by the same method using a similar device as in Reference Example 1, except that oxygen was used instead of air. Using the physiological saline solution containing oxygen nanobubbles according to this embodiment, the mean particle size and density of the oxygen nanobubbles were measured with a cryo-transmission electron microscope of Titan Krios (a product of FEI Co., Ltd.) under the same conditions in the same method as described above. FIG. 5 illustrates a photograph of an electron microscope image of an amorphous ice frozen from pure water containing oxygen nanobubbles.

In FIG. 5, the dark contrast attributable to the mean particle size of 3 nm is observed in the area surrounded by a solid circle line near the bottom of the photograph. In addition, the part seen as a line by the continuous connection of the dark contrast is observed in the area surrounded by a dotted circle line near the center of the photograph, which also indicates the oxygen nanobubbles. From this result it has been clarified that oxygen nanobubbles with the mean particle size of 3 nm were partially aggregated rather than isolated. It was found that 360 oxygen nanobubbles were contained in the amorphous ice volume of 1.8 × 10⁻¹⁴ cc (300 nm×300 nm×200 nm thickness). Because the oxygen nanobubble water was observed using 100-fold diluted water, the density of oxygen nanobubbles in the nanobubble water was evaluated to be 2 × 10¹⁸ bubbles/cc(ml) (200 quadrillion/cc(ml)).

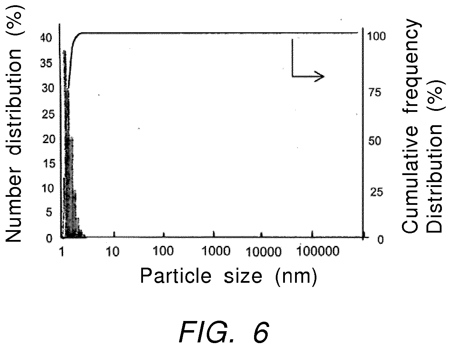

For reference, FIG. 6 illustrates a number distribution of bubble particle sizes of the oxygen nanobubble water in this embodiment, which was measured by a dynamic light scattering method using a distribution and molecular weight measuring system (ELSZ-2000 S) provided as a product of Otsuka Electronics Co., Ltd.. The number distribution shown in FIG. 6 is based on the measurement result obtained after performing the specific data processing as described above. As shown in FIG. 6, the oxygen nanobubbles of the present embodiment are distributed in a narrow range of particle sizes from 1 to 10 nm. The particle size distribution of oxygen nanobubbles is similar to the result shown in FIG. 5. In addition, it is easily seen that the mean particle size is close to 3 nm. Thus, the measurement method of the present invention using the cryo-transmission electron microscope by the ice-embedding method can be also be confirmed to accurately measure the bubble particle of the oxygen nanobuble water by comparison with the dynamic light scattering method.

A physiological saline solution in this embodiment was prepared by blending sodium chloride at a content of 0.9 % by mass based on 100 parts by mass of the physiological saline solution in the water containing oxygen nanobubbles prepared in this manner. This saline solution was used as Embodiment 1.

### < Comparative Example 1>

A physiological saline solution of Comparative Example 1 was prepared by blending sodium chloride in the pure water prior to the generation of the air nanobubbles discussed in Reference 1 above, wherein the content of sodium chloride was 0.9 % by mass based on 100 parts by mass of the normal physiological saline solution.

The cell culture solution containing oxygen nanobubbles (Embodiment 1) and the cell culture solution without oxygen nanobubbles (Comparative Example 1) prepared as described above were used to quantitatively and qualitatively measure changes in cell morphology and the damage level of cells under the anaerobic (hypoxic) stimulation to verify the degree of cell protection. The validation was performed as follows.

Cell culture solutions were prepared using the physiological saline solutions of Embodiment 1 and Comparative Example 1. In the resulting cell culture solutions, vascular smoothness muscle cells were cultured in the anaerobic environment for 12 hours. The morphological changes in the cells were photographed before and after incubation in the anaerobic environment and compared between both. In addition, the amount of LDH was measured before and after the cells were placed under the anaerobic environment, because an amount of enzyme (LDH) that leaks out during cytotoxicity is high in case of damaged cells. LDH means that the higher the amount level thereof is, the higher the cytotoxicity is. In addition, to compare a mitochondrial function (MTT Assay) between Embodiment 1 and Comparative Example 1, cellular growth (cell viability) was measured before and after exposures to anaerobic conditions. The cell viability indicates that the lower the level thereof, the higher the disorder.

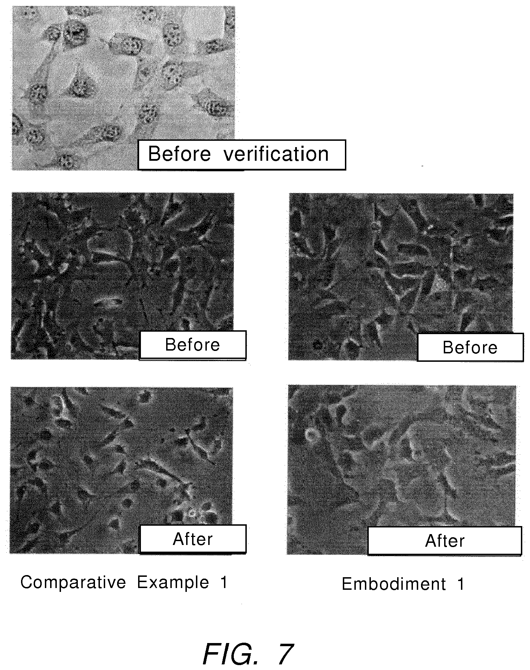

FIGS. 7, 8 and 9 show each measurement result of photographing the morphological changes of the cells, of the amount of LDH, and of the cell viability in the physiological saline solutions of Embodiment 1 and Comparative Example 1, respectively. In FIG. 7, "Before verification" shows the form of vascular smoothness muscle cells found in a normal cell culture solution. In the same figure, "Comparative Example 1 (Before)" and "Embodiment 1 (Before)" mean the morphology before being placed under the anaerobic atmosphere, while "Comparative Example 1 (After)" and "Example 1 (After)" mean the morphology after being left under the anaerobic atmosphere for 12 hours, respectively.

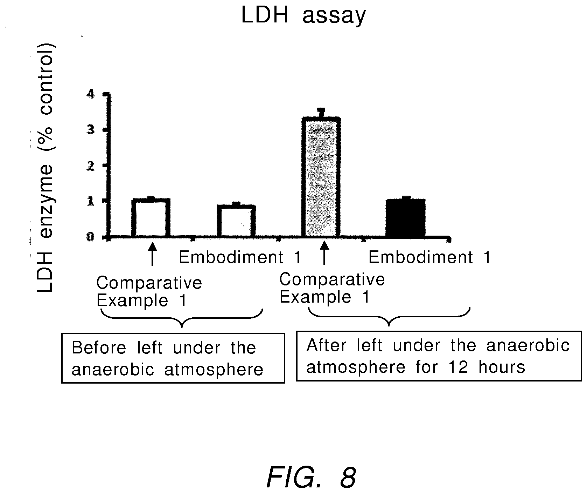

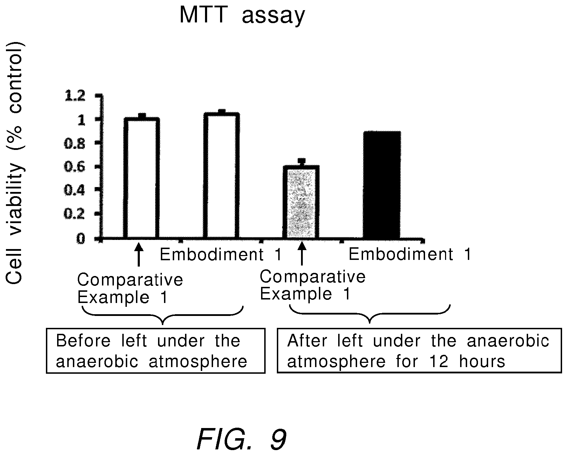

As shown in FIG. 7, Embodiment 1 retained the cell morphology even after 12 hours of incubation under the anaerobic atmosphere, while Comparative Example 1 showed the behavior that the cells were small and dying. Further, as can be seen from the measurement results of the LDH amount shown in FIG. 8, the LDH outflow amount of Embodiment 1 was predominantly reduced after the LDH was left under the anaerobic atmosphere for 12 hours, compared to that of Comparative Example 1. With respect to the values of the cell viability shown in FIG. 9, Embodiment 1 also shows that the difference between the values before and after 12 hours of exposure under the anaerobic atmosphere were smaller than that in Comparative Example 1, indicating that a mitochondrial function was retained.

These results confirm that Embodiment 1 (the cell culture solution containing oxygen nanobubbles) had a very high function in protecting cells and mitochondrial from the anaerobic (hypoxic) stimulation in vascular smoothness muscle cells. In the above verification, the vascular smoothness muscle cells were used as an example. However, it is inferred that the same results can be obtained for all cells, not just for the vascular smooth muscle cells. For example, the physiological saline solution containing the oxygen nanobubbles according to the present invention can reduce cell damages and injuries in the hypoglycemic state, Therefore, an effect of reducing cytotoxicity in the hypoglycemic state during or after exercise can be expected. In addition, the physiological saline solution containing the oxygen nanobubbles according to the present invention can reduce the cell damages and injuries in the hypoxic condition, and thus it is expected to have a protective effect against cytotoxicity caused by myocardial infarction, cerebral infarction, and other circulating perfusion disorders.

### < Comparative Example 2>

An aqueous solution containing oxygen nanobubbles with an air bubble size of 50 to 500 nm was prepared according to the methods disclosed in Patent Literature 2 above. This aqueous solution was used for preparing a cell culture solution. This cell culture solution was used as Comparative Example 2. In order to confirm the cytotoxicity in the cell incubate solution of Comparative Example 2, blood vessel smoothness muscle was first cultured overnight under the normal environment. A conventional cell culture solution and the cell culture solution containing oxygen nanobubbles prepared in Embodiment 1 above were used for comparison. As an index of cytotoxicity, changes in cell morphology and the degree of cell damage were measured qualitatively and quantitatively to verify the level of cell protection, and to compare the degree of cell protection with that in the physiological saline solution in Embodiment 1 above. Validation was performed in the same method as Embodiment 1. The cell viability was measured after left under the anaerobic atmosphere as a test (MTT assay) to measure mitochondrial function, along with observations of morphological changes in cells after left under the anaerobic atmosphere.

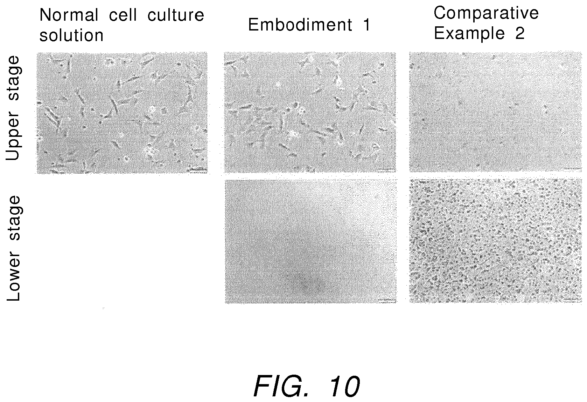

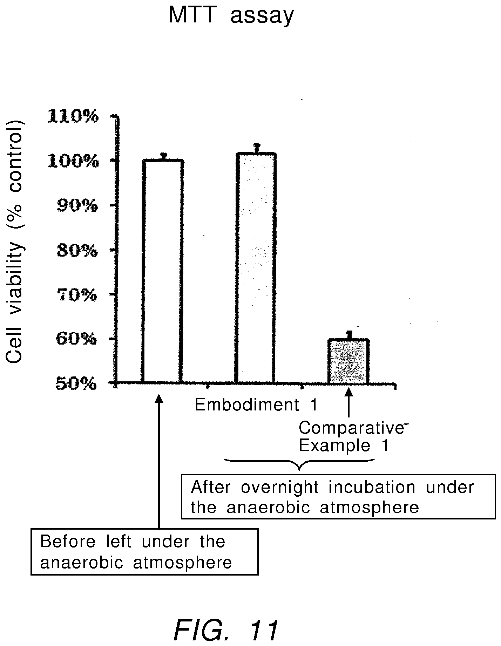

FIGS. 10 and 11 show the results of photographing the morphological changes of the cells in the cell culture solution of Embodiment 1 and Comparative Example 2, and the measurement results of the cell viability by MTT assay, respectively. In FIG. 10, the photograph of "Normal cell culture solution" shown on the left side of the top row is the cell culture solution that does not contain oxygen nanobubbles in the condition before being placed under the anaerobic atmosphere. In Fig. 10, the upper center and the upper right side are photos showing the morphological changes of the cells after overnight incubation under the anaerobic atmosphere. On the other hand, in Fig. 10, the middle and right sides of the lower row are photos showing changes in the culture medium after overnight exposure under the anaerobic atmosphere, using only culture solutions without containing cells.

As can be seen from the photograph shown at the top of FIG. 10, Embodiment 1 retained cell morphology even after overnight incubation under the anaerobic atmosphere, and no cytotoxicity was observed in the culture medium. On the other hand, Comparative Example 2 (the cell culture solution having the aqueous solution containing an oxygen nanobubbles with an air bubble size of 50 to 500 nm) showed very high cytotoxicity, and almost all cells were killed after overnight exposure. In addition, in the photos of the culture solution only shown in the lower part of FIG. 10, it was observed that Embodiment 1 had no sediment, while Comparative Example 2 had many deposits in the culture solution. Thus, it is inferred that the culture solution of Comparative Example 2 resulted in the observation of cytotoxicity by agglutination of proteins therein. This result can also be readily understood from the results of the cell viability measurements shown in FIG. 11. That is, Embodiment 1 showed no change in the cell survival after overnight incubation under the anaerobic atmosphere, whereas Comparative Example 2 showed a large decrease in the cell viability. From the above results, it has been confirmed that the use of the physiological saline solution according to the present invention, which contains a large amount of oxygen nanobubbles having the mean particle size of 30 nm or less, preferably 1 to 10 nm, at a density of 1 × 10¹⁶ bubbles or more, preferably 1 × 10¹⁷ bubbles or more, reduces cytotoxicity and can be safely administered to a living body for the first time.

### <Embodiment 2>

Excepting that an aqueous solution containing sodium chloride at a content of 0.85 to 0.95% by mass was used, a physiological saline solution containing oxygen nanobubbles were prepared under the same conditions by the same method using the same device as in Embodiment 1. This solution was used as a physiological saline solution without an addition of sodium chloride after generating oxygen nanobubbles. The physiological saline solution containing oxygen nanobubbles according to this embodiment was confirmed the oxygen nanbubbles to have the mean particle size of 3 nm and the density of 2 × 10¹⁸ bubbles/cc (ml), respectively, by the measurement with the cryo-transmission electron microscope Titan Krios (manufactured by FEI Co., Ltd.) using the same conditions and the methods as described above. The physiological saline solution of this embodiment was used as Embodiment 2.

The cell culture solution prepared using the physiological saline solution of Embodiment 2 obtained in this manner was used to quantitatively and qualitatively measure changes in cell morphology and the degree of cell damage under the anaerobic (hypoxic) stimulation to verify the level of cell protection, and compare the level of cell protection with the physiological saline solution of Embodiment 1. Validation was performed in the same method as Embodiment 1. The amounts of enzymes (LDHs) leaked out during the cell disorder and the values of cell growth (cell viability) were measured, as well as observations of the changes in cell morphology before and after left in the anaerobic condition.

The validation results in the cell culture solution of Embodiment 2 are similar to those in Embodiment 1, indicating no cytotoxicity. Thus, it was found that the cell culture medium prepared using the physiological saline according to the present invention was not affected by the addition of sodium chloride before and after the step of generating oxygen nanobubbles, and it was confirmed that the similar effect of protecting cells under the anaerobic (hypoxic) stimulation was obtained in the both cases.

### <Embodiment 3>

In this embodiment, the administrable aqueous solution to a living body according to the present invention was used to verify the effect of inhibiting or preventing the proliferation and hyperplasia of cancer cells. A cell culture solution containing a serum and a culture medium was concretely prepared using the aqueous solution containing oxygen nanobubbles with a mean particle size of 3 nm, produced in the same method as described in Embodiment 3 above. The generation extent of the protein (HIF-1a) induced by a lung cancer cell was measured when the lung cancer cells of the lung squamous cell line EBC1 were injected into a glass well plate for the cell culture, and then were placed therein under the hypoxic condition for a predetermined time.

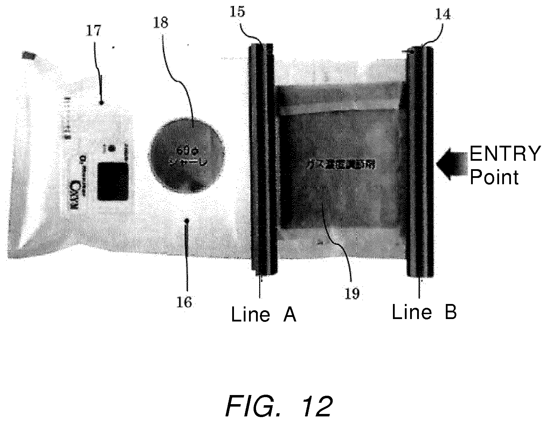

FIG. 12 illustrates a commercially available hypoxic culture kit used when cancer cells are cultured. As illustrated in FIG. 12, the hypoxic culture kit is provided with clips 14, 15 along each line of lines A and B, and the internal of the gas barrier pouch bag 16 can be divided into two spaces to keep each space closed by clips 14, 15. The O₂ sensor 17 is set behind line A, and a cell culture well plate 18 filled with a culture solution is placed just beside the O₂ sensor 17. A gas concentration regulator 19 is placed between line A and line B. The gas concentration regulator 19 is opened, and taken out from the aluminum bag during use, and is placed near the ENTRY point from line A and sealed using the clip 14 provided in line B. This results in short-term absorption of O₂ within the gas barrier pouch bag 16, creating a hypoxic environment. The O₂ concentration inside the gas barrier pouch bag 16 monitors the scale of the O₂ sensor 17 and the air inside the cell culture well plate 18 is replaced with the air inside the gas barrier pouch bag 16 when the desired O₂ concentration is approached. And, when the O₂ concentration is slightly lower than the desired value, the gas barrier pouch bag is blocked by a clip 15 provided in line A to stop the absorption of O₂. The concentration of O₂ in the space between line A and line B can be adjusted using a clip 15 provided in line A. In this embodiment, the O₂ concentration in the space between line A and line B was adjusted to be about 1%.

The generation extent of protein (HIF-1a) induced by the lung cancer cells was determined by a Western blotting (Western Blotting) technique when placed under the hypoxic atmosphere. The western blotting method represents a technique that combines the excellent electrophoretic resolution with the high specificity for antigen-antibody reactions to detect specific proteins from a mixture of proteins, which is known as a measurement method used for protein detection and analysis. In this embodiment, in addition to the protein (HIF-1a) induced by the lung cancer cells, the b-actin protein contained in the pulmonary squamous cell carcinoma cell line EBC1 was also measured as a control of the protein generation to ensure that the sample in the cell culture well plates contains proteins certainly and that the total protein content used in the Western blotting method is constant.

### < Comparative Example 3>

Excepting that an aqueous solution containing no oxygen nanobubbles was used instead of the aqueous solution containing oxygen nanobubbles according to Embodiment 3 , the generation extent of the protein (HIF-1a) induced by the lung cancer cells was verified in the same manner as in Embodiment 3. The generation extent of the protein (HIF-1a) induced by the lung cancer cells was measured by the Western blotting method as described in Embodiment 3. This verification example was referred to as Comparative Example 3.

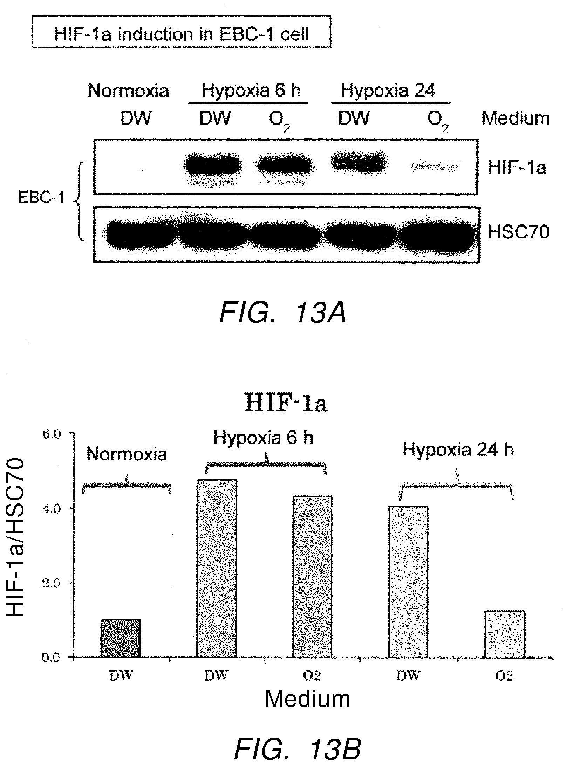

FIGS. 13A and 13B show the induction results of HIF-1a and HSC70 proteins for Embodiment 3 and Comparative Example 3, respectively, after leaving each of the solutions under a hypoxic atmosphere with O₂ concentrations of about 1% for 6 and 24 hours. The [Normoxia], [Hypoxia 6 h], and [Hypoxia 24 h] shown in FIGS. 13A and 13B respectively mean each sample before placed in the low oxygen atmosphere, after left for 6 hours therein, and after left for 24 hours therein. "O₂" and "DW" displayed on [Medium] in the figures mean Embodiment 3 and Comparative Example 3, respectively. In figures, the vertical columns in "O₂" and "DW" show the generation results of proteins measured in each of their validated samples.

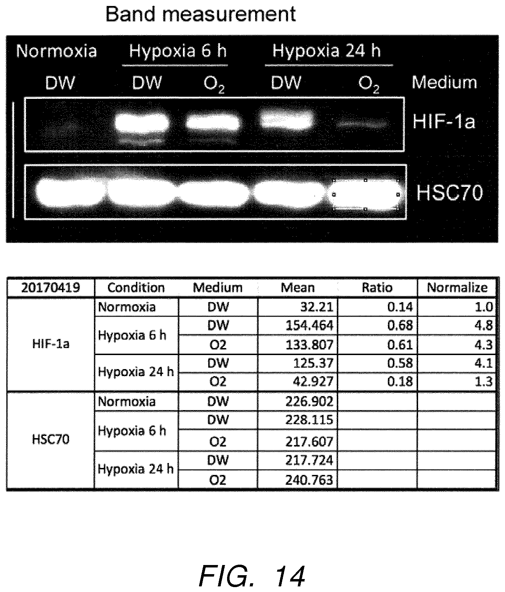

FIG. 13A illustrates the assay results measured by the Western blotting method in which the black portions in the figure represent the generation of HIF-1a and HSC70 proteins. The darker the black portion in the figure, the stronger the generating degree of protein is. FIG. 13B shows the area ratios of the black portion of HIF-1a to that of HSC70 in each sample, which were obtained by calculating each area of the black portions shown in FIG. 13A. The areas of the black portions shown in FIG. 13A can be numerically converted by performing image processing, as shown in FIG. 14. In FIG. 14, for example, focusing on the state (Normoxia) before left under the hypoxic atmosphere, the generation of HIF-1a was rarely observed and the area of the black portion was counted to be 32.21 as the mean value (as shown in "Mean" column), whereas the black portion attributed to the generation of HSC70 was counted to be 226.902 as the mean value (as shown in "Mean" column) , indicating that the area ratio of HIF-1a to HSC70 (shown in "Ratio" column ) is 0.14. Here, it can be seen that the generation of HSC70 is almost constant without substantial changes before and left under the hypoxic atmosphere. Furthermore, the same procedure was performed for Embodiment 3 (O₂) and Comparative Example 3 (DW) after left under the hypoxic atmosphere for 6 and 24 hours. The area ratios of the black portions attributed to the generation of HIF-1a to that of HSC70 were obtained by calculating the area ratios (Ratio) and normalizing them (see Normalize), assuming that the Normoxia case is 1. The result shown in the lower side of FIG. 14 represents the normalized area ratios of HIF-1a to HSC70.

As shown in FIG. 14, when normalized the area ratios assuming that the Normoxia case is 1, any large difference in the generation of HIF-1a between Example 3 (O₂) and Embodiment 3 (DW) was not observed after left under the hypoxic condition for 6 hours (Hypoxia 6h). On the other hand, a large difference in the inhibitory effect on the generation of HIF-1a between both was observed in the longer-term placement after left under the hypoxic atmosphere (Hypoxia 24 h), indicating the generation of HIF-1a to be significantly depressed in Embodiment 3 compared to Comparative Example 3. This suggests that the 6 hours placement under the hypoxic atmosphere only showed a transient phenomenon in which the effect of the oxygen nanobubbles was not substantially observed due to the short placement time. However, the results of the Hypoxia 24 h indicates that the effect of the oxygen nanobubbles was increased with the increase of the placement time. Although not shown in Figure 13, the test was performed in a similar method when left in the hypoxic atmosphere for 48 hours. The results confirmed that the inhibitory effect was equivalent to or greater than that in 24 hours placement. Therefore, it can be inferred that the aqueous solution containing oxygen nanobubbles sufficiently has an effect of suppressing the proliferation of cancer cells.

As described above, the administrable aqueous solution to the living body according to the present invention has the function to sufficiently supply oxygen to peripheral cells, resulting in enhancement of the effect of preventing cells due to less damage and injury of the cells under the hypoxic or anaerobic stimulation and the effect of suppressing or preventing the proliferation and hypertrophy of cancer cells that are likely to occur in the anaerobic environment. Furthermore, the stable effect of protecting cells can be obtained over the long term, and the variation of its effect can be remarkably reduced. Therefore, the aqueous solution according to the present invention can be safely administered to a living body or ingested orally.

In the method of manufacturing the administrable aqueous solution to a living body according to the present invention, the oxygen nanobubbles having a smaller particle size can be also generated in large quantities and stably compared to conventional methods for generating nanobubbles. The method can also produce the administrable aqueous solution to a living body, by which the effect of preventing cells under the hypoxic or anaerobic stimulation, and the effect of suppressing or preventing the proliferation and hypertrophy of cancer cells that are likely to occur in the anaerobic environment, can be stably obtained over the long term. The aqueous solution containing oxygen nanobubbles that has such effects was hardly manufactured in the prior arts, and can be obtained for the first time by the manufacturing method of the present invention.

### Industrial applicability

Thus, the administrable aqueous solution to a living body according to the present invention can protect cells under the hypoxic or anaerobic stimulation, and suppress or prevent the proliferation and hypertrophy of cancer in such the same environment by being administrated or orally ingested as a physiological saline solution or an infusion solution, or using as a cell culture solution. Therefore, the administrable aqueous solution according to the present invention has extremely high utility in various medical fields.

## Claims

1. An administrable aqueous solution to a living body,
the administrable aqueous solution comprising an aqueous solution containing oxygen nanobubbles having a mean particle size of 30 nm or less and a density of 10₁₆ bubbles or more per 1ml of the aqueous solution,
wherein the mean particle size and the density of the oxygen nanobubbles are determined by a measurement with a cryo-transmission electron microscope using an ice-embedded method, **characterized in that**
the administrable aqueous solution is for use in a method of inhibiting or preventing the proliferation and hypertrophy of cancers, wherein the method comprises the administration or oral ingestion of the administrable aqueous solution to the living body..

2. The administrable aqueous solution to a living body according to claim 1,
the administrable aqueous solution comprising an aqueous solution containing oxygen nanobubbles having a mean particle size of 1 to 10 nm and a density of 10₁₇ bubbles or more per 1 ml of the aqueous solution,
wherein the mean particle size and the density of the oxygen nanobubbles are determined by the measurement with the cryo-transmission electron microscope using the ice-embedded method.

3. The administrable aqueous solution to a living body according to claim 1 or claim 2, wherein the administrable aqueous solution is a physiological saline solution comprising: the aqueous solution containing oxygen nanobubbles; and sodium chloride of 0.85 to 0.95 % by mass based on 100 parts by mass of the physiological saline solution.

4. The administrable aqueous solution to a living body according to any of claims 1 to 3, wherein the administrable aqueous solution is an infusion solution selected from the group consisting of hypotonic composite electrolyte solutions supplemented with 5 mass% of glucose solution, Ringer's solutions, and physiological saline solutions with a high-calorie fluid and heparin, which comprise the aqueous solution containing oxygen nanobubbles.

5. The administrable aqueous solution to a living body according to any of claims 1 to 4, wherein the administrable aqueous solution is a cell culture solution comprising the aqueous solution containing oxygen nanobubbles.

6. A method for manufacturing an administrable aqueous solution to a living body, the method comprising:
producing jets of an aqueous solution containing dissolved-oxygen by injecting the aqueous solution from an outside of a cylinder via two or more small through-holes in the cylinder, at a pressure higher than the atmospheric pressure, the through-holes being arranged in a circumferential direction thereof with such a configuration that the respective openings of the two or more small through-holes are arranged facing each other on the same plane parallel to the radial cross section of the cylinder;
creating a collision of the jets of the aqueous solution inside the cylinder so as that a water hammer of the jets concentrates at the center thereof; and
generating oxygen nanobubbles by the mutual collision of the jets of the aqueous solution,
wherein the oxygen nanobubbles have a mean particle size of 30 nm or less and a density of 10₁₆ bubbles or more per 1 ml of the aqueous solution,
wherein the mean particle size and the density of the oxygen nanobubbles are determined by a measurement with a cryo-transmission electron microscope using an iceembedded method, and
wherein the administrable aqueous solution is a cell culture solution for culturing cells, or an aqueous solution for inhibiting or preventing proliferation and hypertrophy of cancers.

7. The method for manufacturing the administrable aqueous solution to a living body according to claim 6,
wherein the oxygen nanobubbles have a mean particle size of 1 to 10 nm and a density of 10₁₇ bubbles or more per 1ml of the aqueous solution,
wherein the mean particle size and the density of the oxygen nanobubbles are determined by the measurement with the cryo-transmission electron microscope using the ice-embedded method.

8. The method for manufacturing the administrable aqueous solution to a living body according to claim 6 or claim 7,
wherein the oxygen bubbles are generated by a bubble generation means, the bubble generation means comprising:
a means for sucking each of oxygen and liquid;
a means for pressurizing the oxygen and the liquid in a lump and transferring them;
an oxygen-liquid mixing vessel for enriching the dissolved oxygen by mixing the transferred liquid, which includes the oxygen, with another new oxygen; and
a means for generating nanobubbles using the dissolved liquid of the oxygen-liquid mixing state prepared in the oxygen-liquid mixing vessel, wherein the means has a bubble generating nozzle for generating nanobubbles, using a water hammering power caused by the mutual collision of the dissolved liquid with oxygen,
wherein the bubble generating nozzle comprises:
the cylinder having two or more small through-holes, arrayed in the circumferential direction thereof with such a configuration that the respective opening of each of such two or more small through-holes faces each other in the same plane; and a nanobubble discharge port provided on at least one end of the hollow cylinder,
wherein the small through-holes are arranged so as that all of their extension lines passing through respective center of the cross-section of each of the small through-holes intersect each other in the inside of the hollow of the cylinder.

9. The method for manufacturing the administrable aqueous solution to a living body according to any of claims 6 to 8,
wherein four to eight numbers of the small through-holes are equidistantly arranged in the circumferential direction of the cylinder, facing each on a same plane parallel to a radial cross-section parallel of the cylinder, and the pore diameter of the portion leading to the hollow of the cylinder is 0.1 to 0.5 mm.

10. The method for manufacturing the administrable aqueous solution to a living body according to any of claims 6 to 9,
wherein the administrable aqueous solution is a physiological saline solution prepared by adding sodium chloride to the aqueous solution containing oxygen nanobubbles at a content of 0.85-0.95 % by mass based on 100 parts by mass of the physiological saline solution.

11. The method for manufacturing the administrable aqueous solution to a living body according to claim 10,
wherein the administrable aqueous solution is a physiological saline solution manufactured by using an aqueous solution containing 0.85 to 0.95% by mass of sodium chloride based on 100 parts by mass of the aqueous solution, as the dissolved liquid of the oxygen-liquid mixing state.

12. The method for manufacturing the administrable aqueous solution to a living body according to claim 10, the method comprising:
preparing the aqueous solution containing oxygen nanobubbles using an aqueous solution having no sodium chloride as the dissolved liquid of the oxygen-liquid mixing state; and
manufacturing the physiological saline solution by adding sodium chloride to the aqueous solution containing oxygen nanobubbles at the content of 0.85 to 0.95% by mass based on 100 parts by mass of the physiological saline solution.

13. The method for manufacturing the administrable aqueous solution to a living body according to any of claims 6 to 12,
wherein the administrable aqueous solution is an infusion solution manufactured by adding at least an additive to the administrable aqueous solution, wherein the additive contains at least one selected from the group consisting of at least one element of potassium and calcium, 5% glucose solution, an amino acid, and heparin.

14. The method for manufacturing the administrable aqueous solution to a living body according to any of claims 6 to 13,
wherein the administrable aqueous solution is manufactured as a cell culture solution for culturing cells.

15. The method for manufacturing the administrable aqueous solution to a living body according to any of claims 6 to13,
wherein the administrable aqueous solution is manufactured as an aqueous solution used by an administration or an oral ingestion to the living body to inhibit or prevent proliferation and hypertrophy of cancers.

## Patentansprüche

1. Einem lebenden Körper verabreichbare wässrige Lösung,
wobei die verabreichbare wässrige Lösung eine wässrige Lösung umfasst, die Sauerstoff-Nanoblasen umfasst, die eine mittlere Partikelgröße von 30 nm oder weniger und eine Dichte von 10₁₆ Blasen pro 1 ml oder mehr der wässrigen Lösung aufweisen,
wobei die mittlere Partikelgröße und die Dichte der Sauerstoff-Nanoblasen durch eine Messung mit einem Kryoelektronenmikroskop unter Verwendung eines Eis-eingebetteten Verfahrens bestimmt werden, **dadurch gekennzeichnet, dass**
die verabreichbare wässrige Lösung zur Verwendung in einem Verfahren zur Hemmung oder Verhinderung der Proliferation und Hypertrophie von Krebsarten dient, wobei das Verfahren die Verabreichung der verabreichbaren wässrigen Lösung an den lebenden Körper oder die orale Einnahme umfasst.

2. Einem lebenden Körper verabreichbare wässrige Lösung nach Anspruch 1,
wobei die verabreichbare wässrige Lösung eine wässrige Lösung umfasst, die Sauerstoff-Nanoblasen umfasst, die eine mittlere Partikelgröße von 1 bis 10 nm und eine Dichte von 10₁₇ Blasen pro 1 ml oder mehr der wässrigen Lösung aufweisen,
wobei die mittlere Partikelgröße und die Dichte der Sauerstoff-Nanoblasen durch die Messung mit dem Kryoelektronenmikroskop unter Verwendung des Eis-eingebetteten Verfahrens bestimmt werden.

3. Einem lebenden Körper verabreichbare wässrige Lösung nach Anspruch 1 oder Anspruch 2,
wobei die verabreichbare wässrige Lösung eine physiologische Kochsalzlösung ist, die umfasst: die wässrige Lösung, die Sauerstoff-Nanoblasen enthält; und bezogen auf 100 Masseteile der physiologischen Kochsalzlösung Natriumchlorid von 0,85 bis 0,95 Masse-%.

4. Einem lebenden Körper verabreichbare wässrige Lösung nach einem der Ansprüche 1 bis 3,
wobei die verabreichbare wässrige Lösung eine Infusionslösung ist, die aus der Gruppe ausgewählt ist, bestehend aus hypotonischen Kompositelektrolytlösungen, ergänzt mit 5 Masse-% Glukoselösung, Ringer-Lösungen und physiologischen Kochsalzlösungen mit einem hochkalorischen Fluid und Heparin, die die Sauerstoff-Nanoblasen enthaltende wässrige Lösung umfassen.

5. Einem lebenden Körper verabreichbare wässrige Lösung nach einem der Ansprüche 1 bis 4,
wobei die verabreichbare wässrige Lösung eine Zellkulturlösung ist, die die Sauerstoff-Nanoblasen enthaltende wässrige Lösung umfasst.

6. Verfahren zum Herstellen einer einem lebenden Körper verabreichbaren wässrigen Lösung, wobei das Verfahren umfasst:
Erzeugen von Strahlen einer wässrigen Lösung, die gelösten Sauerstoff enthält, durch Einspritzen der wässrigen Lösung von einer Außenseite eines Zylinders über zwei oder mehr kleine Durchgangslöcher in dem Zylinder mit einem Druck, der höher als der Atmosphärendruck ist, wobei die Durchgangslöcher in einer Umfangsrichtung davon mit einer derartigen Konfiguration angeordnet sind, dass die jeweiligen Öffnungen der zwei oder mehr kleinen Durchgangslöcher einander zugewandt auf derselben Ebene parallel zum radialen Querschnitt des Zylinders angeordnet sind;
Erzeugen einer Kollision der Strahlen der wässrigen Lösung im Inneren des Zylinders, sodass sich ein Wasserschlag der Strahlen in dessen Mitte konzentriert; und
Erzeugen von Sauerstoff-Nanoblasen durch die gegenseitige Kollision der Strahlen der wässrigen Lösung,
wobei die Sauerstoff-Nanoblasen eine mittlere Partikelgröße von 30 nm oder weniger und eine Dichte von 10₁₆ Blasen pro 1 ml oder mehr der wässrigen Lösung aufweisen,
wobei die mittlere Partikelgröße und die Dichte der Sauerstoff-Nanoblasen durch eine Messung mit einem Kryoelektronenmikroskop unter Verwendung eines Eis-eingebetteten Verfahrens bestimmt werden, und
wobei die verabreichbare wässrige Lösung eine Zellkulturlösung zum Kultivieren von Zellen oder eine wässrige Lösung zum Hemmen oder Verhindern von Proliferation und Hypertrophie von Krebsarten ist.

7. Verfahren zum Herstellen der einem lebenden Körper verabreichbaren wässrigen Lösung nach Anspruch 6,
wobei die Sauerstoff-Nanoblasen eine mittlere Partikelgröße von 1 bis 10 nm und eine Dichte von 10₁₇ Blasen pro 1 ml oder mehr der wässrigen Lösung aufweisen,
wobei die mittlere Partikelgröße und die Dichte der Sauerstoff-Nanoblasen durch die Messung mit dem Kryoelektronenmikroskop unter Verwendung des Eis-eingebetteten Verfahrens bestimmt werden.

8. Verfahren zum Herstellen der einem lebenden Körper verabreichbaren wässrigen Lösung nach Anspruch 6 oder Anspruch 7,
wobei die Sauerstoffblasen durch ein Blasenerzeugungsmittel erzeugt werden, wobei das Blasenerzeugungsmittel umfasst:
ein Mittel zum Ansaugen von jeweils Sauerstoff und Flüssigkeit;
ein Mittel, um den Sauerstoff und die Flüssigkeit in einem unter Druck zu setzen und diese zu übertragen;
ein Sauerstoff-Flüssigkeit-Mischgefäß zum Anreichern des gelösten Sauerstoffs durch Mischen der übertragenen Flüssigkeit, die den Sauerstoff enthält, mit anderem neuen Sauerstoff; und
ein Mittel zum Erzeugen von Nanoblasen unter Verwendung der gelösten Flüssigkeit im Sauerstoff-Flüssigkeit-Mischzustand, die im Sauerstoff-Flüssigkeit-Mischgefäß präpariert wurde, wobei das Mittel eine Blasenerzeugungsdüse zum Erzeugen von Nanoblasen unter Verwendung einer Wasserschlagkraft aufweist, die durch die gegenseitige Kollision der gelösten Flüssigkeit mit Sauerstoff verursacht wird,
wobei die Blasenerzeugungsdüse umfasst:
den Zylinder, der zwei oder mehr kleine Durchgangslöcher aufweist, die in dessen Umfangsrichtung mit einer derartigen Konfiguration angeordnet sind, dass die jeweiligen Öffnungen eines jeden dieser zwei oder mehr kleinen Durchgangslöcher einander in derselben Ebene zugewandt sind; und eine Nanoblasen-Abgabeöffnung, die an mindestens einem Ende des Hohlzylinders bereitgestellt ist,
wobei die kleinen Durchgangslöcher so angeordnet sind, dass alle ihre Verlängerungslinien, die durch die jeweilige Mitte des Querschnitts jedes der kleinen Durchgangslöcher verlaufen, einander im Inneren des Hohlraums des Zylinders schneiden.

9. Verfahren zum Herstellen der einem lebenden Körper verabreichbaren wässrigen Lösung nach einem der Ansprüche 6 bis 8,
wobei vier bis acht der kleinen Durchgangslöcher mit gleichem Abstand in der Umfangsrichtung des Zylinders einander auf derselben Ebene zugewandt parallel zu einem radialen Querschnitt parallel zum Zylinder angeordnet sind, und der Porendurchmesser des Abschnitts, der zu dem Hohlraum des Zylinders führt, 0,1 bis 0,5 mm beträgt.

10. Verfahren zum Herstellen der einem lebenden Körper verabreichbaren wässrigen Lösung nach einem der Ansprüche 6 bis 9,
wobei die verabreichbare wässrige Lösung eine physiologische Kochsalzlösung ist, die durch Zugeben von Natriumchlorid zu der wässrigen Lösung präpariert wird, die bezogen auf 100 Masseteile der physiologischen Kochsalzlösung Sauerstoff-Nanoblasen mit einem Gehalt von 0,85 bis 0,95 Masse-% enthält.

11. Verfahren zum Herstellen der einem lebenden Körper verabreichbaren wässrigen Lösung nach Anspruch 10,
wobei die verabreichbare wässrige Lösung eine physiologische Kochsalzlösung ist, die unter Verwendung einer wässrigen Lösung, die bezogen auf 100 Masseteile der wässrigen Lösung 0,85 bis 0,95 Masse-% Natriumchlorid enthält, als gelöste Flüssigkeit im Sauerstoff-Flüssigkeit-Mischzustand hergestellt wird.

12. Verfahren zum Herstellen der einem lebenden Körper verabreichbaren wässrigen Lösung nach Anspruch 10, wobei das Verfahren umfasst:
Präparieren der wässrigen Lösung, die Sauerstoff-Nanoblasen enthält, unter Verwendung einer wässrigen Lösung, die kein Natriumchlorid als gelöste Flüssigkeit des Sauerstoff-Flüssigkeit-Mischzustands aufweist; und
Herstellen der physiologischen Kochsalzlösung durch Zugeben von Natriumchlorid zu der wässrigen Lösung, die bezogen auf 100 Masseteile der physiologischen Kochsalzlösung Sauerstoff-Nanoblasen mit dem Gehalt von 0,85 bis 0,95 Masse-% enthält.

13. Verfahren zum Herstellen der einem lebenden Körper verabreichbaren wässrigen Lösung nach einem der Ansprüche 6 bis 12,
wobei die verabreichbare wässrige Lösung eine Infusionslösung ist, die durch Zugeben mindestens eines Zusatzstoffs zu der verabreichbaren wässrigen Lösung hergestellt wird, wobei der Zusatzstoff mindestens eines enthält, das ausgewählt ist aus der Gruppe, bestehend aus mindestens einem Element von Kalium und Calcium, 5%iger Glukoselösung, einer Aminosäure und Heparin.

14. Verfahren zum Herstellen der einem lebenden Körper verabreichbaren wässrigen Lösung nach einem der Ansprüche 6 bis 13,
wobei die verabreichbare wässrige Lösung als Zellkulturlösung zum Kultivieren von Zellen hergestellt wird.

15. Verfahren zum Herstellen der einem lebenden Körper verabreichbaren wässrigen Lösung nach einem der Ansprüche 6 bis 13,
wobei die verabreichbare wässrige Lösung als wässrige Lösung hergestellt wird, die durch Verabreichung an den lebenden Körper oder orale Einnahme verwendet wird, um Proliferation und Hypertrophie von Krebsarten zu hemmen oder zu verhindern.

## Revendications

1. Solution aqueuse administrable à un corps vivant,
la solution aqueuse administrable comprenant une solution aqueuse contenant des nanobulles d'oxygène ayant une taille moyenne de particule de 30 nm ou moins et une densité de 10₁₆ bulles ou plus pour 1 ml de solution aqueuse,
dans laquelle la taille moyenne de particule et la densité des nanobulles d'oxygène sont déterminées par une mesure au microscope électronique à transmission cryogénique selon un procédé d'incorporation de glace, **caractérisée en ce que**
la solution aqueuse administrable est destinée à être utilisée dans un procédé d'inhibition ou de prévention de la prolifération et de l'hypertrophie des cancers, le procédé comprenant l'administration ou l'ingestion orale de la solution aqueuse administrable au corps vivant.

2. Solution aqueuse administrable à un corps vivant selon la revendication 1,
la solution aqueuse administrable comprenant une solution aqueuse contenant des nanobulles d'oxygène ayant une taille moyenne de particule de 1 à 10 nm et une densité de 10₁₇ bulles ou plus pour 1 ml de solution aqueuse,
la taille moyenne de particule et la densité des nanobulles d'oxygène étant déterminées par la mesure au microscope électronique à transmission cryogénique selon le procédé d'incorporation de glace.

3. Solution aqueuse administrable à un corps vivant selon la revendication 1 ou la revendication 2,
la solution aqueuse administrable étant une solution saline physiologique comprenant : la solution aqueuse contenant des nanobulles d'oxygène ; et du chlorure de sodium de 0,85 à 0,95 % en masse sur la base de 100 parties en masse de solution saline physiologique.

4. Solution aqueuse administrable à un corps vivant selon l'une quelconque des revendications 1 à 3,
la solution aqueuse administrable étant une solution pour perfusion choisie dans le groupe constitué de solutions d'électrolytes composites hypotoniques complétées de 5 % en masse de solution de glucose, de solutions de Ringer et de solutions salines physiologiques avec un fluide riche en calories et de l'héparine, qui comprennent la solution aqueuse contenant des nanobulles d'oxygène.

5. Solution aqueuse administrable à un corps vivant selon l'une quelconque des revendications 1 à 4,
la solution aqueuse administrable étant une solution de culture cellulaire comprenant la solution aqueuse contenant des nanobulles d'oxygène.

6. Procédé de fabrication d'une solution aqueuse administrable à un corps vivant, le procédé comprenant :
la production de jets d'une solution aqueuse contenant de l'oxygène dissous par injection de la solution aqueuse depuis l'extérieur d'un cylindre via deux petits trous traversants ou plus dans le cylindre, à une pression supérieure à la pression atmosphérique, les trous traversants étant disposés dans leur direction circonférentielle dont la configuration permet aux ouvertures respectives des deux petits trous traversants ou plus de se faire face sur le même plan parallèle à la section transversale radiale du cylindre ;
la création d'une collision des jets de la solution aqueuse à l'intérieur du cylindre de manière à ce qu'un coup de bélier des jets se concentre au centre de celui-ci ; et
la génération de nanobulles d'oxygène par collision mutuelle des jets de la solution aqueuse,
dans lequel les nanobulles d'oxygène ont une taille moyenne de particule de 30 nm ou moins et une densité de 10₁₆ bulles ou plus pour 1 ml de solution aqueuse,
dans lequel la taille moyenne de particule et la densité des nanobulles d'oxygène sont déterminées par une mesure avec un microscope électronique à transmission cryogénique selon un procédé d'incorporation de glace, et
dans lequel la solution aqueuse administrable est une solution de culture cellulaire pour cultiver des cellules, ou une solution aqueuse pour inhiber ou prévenir la prolifération et l'hypertrophie des cancers.

7. Procédé de fabrication de la solution aqueuse administrable à un corps vivant selon la revendication 6,
dans lequel les nanobulles d'oxygène ont une taille moyenne de particule de 1 à 10 nm et une densité de 10₁₇ bulles ou plus pour 1 ml de solution aqueuse,
dans lequel la taille moyenne de particule et la densité des nanobulles d'oxygène sont déterminées par la mesure au microscope électronique à transmission cryogénique selon le procédé d'intégration de glace.

8. Procédé de fabrication de la solution aqueuse administrable à un corps vivant selon la revendication 6 ou la revendication 7,
dans lequel les bulles d'oxygène sont générées par un moyen de génération de bulles, le moyen de génération de bulles comprenant :
un moyen pour aspirer de l'oxygène et du liquide ;
un moyen pour mettre sous pression l'oxygène et le liquide en bloc et les transférer
un récipient de mélange oxygène-liquide pour enrichir l'oxygène dissous en mélangeant le liquide transféré, qui comprend l'oxygène, avec un autre nouvel oxygène ; et
un moyen pour générer des nanobulles à l'aide du liquide dissous de l'état de mélange oxygène-liquide préparé dans le récipient de mélange oxygène-liquide, le moyen comportant une buse de génération de bulles pour générer des nanobulles, à l'aide d'une force de coup de bélier provoquée par la collision mutuelle du liquide dissous et de l'oxygène,
dans lequel la buse de génération de bulles comprend :
le cylindre ayant au moins deux petits trous traversants, disposés dans sa direction circonférentielle dont la configuration permet aux ouvertures respectives de chacun de ces au moins deux petits trous traversants de se faire face dans le même plan ; et un orifice d'évacuation de nanobulles disposé sur au moins une extrémité du cylindre creux,
dans lequel les petits trous traversants sont disposés de telle sorte que toutes leurs lignes de rappel passant par le centre respectif de la section transversale de chacun des petits trous traversants se croisent à l'intérieur du creux du cylindre.

9. Procédé de fabrication de la solution aqueuse administrable à un corps vivant selon l'une quelconque des revendications 6 à 8,
dans lequel quatre à huit petits trous traversants sont disposés à égale distance dans la direction circonférentielle du cylindre, chacun se faisant face sur un même plan parallèle à une section transversale radiale parallèle du cylindre, et le diamètre des pores de la partie menant au creux du cylindre est de 0,1 à 0,5 mm.

10. Procédé de fabrication de la solution aqueuse administrable à un corps vivant selon l'une quelconque des revendications 6 à 9,
dans lequel la solution aqueuse administrable est une solution saline physiologique préparée par ajout de chlorure de sodium à la solution aqueuse dont la teneur en nanobulles d'oxygène est de 0,85 à 0,95 % en masse sur la base de 100 parties en masse de la solution saline physiologique.

11. Procédé de fabrication de la solution aqueuse administrable à un corps vivant selon la revendication 10,
dans lequel la solution aqueuse administrable est une solution saline physiologique fabriquée à l'aide d'une solution aqueuse contenant 0,85 à 0,95 % en masse de chlorure de sodium sur la base de 100 parties en masse de la solution aqueuse, comme liquide dissous à l'état de mélange oxygène-liquide.

12. Procédé de fabrication de la solution aqueuse administrable à un corps vivant selon la revendication 10, le procédé comprenant :
la préparation de la solution aqueuse contenant des nanobulles d'oxygène à l'aide d'une solution aqueuse ne contenant pas de chlorure de sodium comme liquide dissous à l'état de mélange oxygène-liquide ; et
la fabrication de la solution saline physiologique par ajout de chlorure de sodium à la solution aqueuse contenant des nanobulles d'oxygène dont la teneur est de 0,85 à 0,95 % en masse sur la base de 100 parties en masse de la solution saline physiologique.

13. Procédé de fabrication de la solution aqueuse administrable à un corps vivant selon l'une quelconque des revendications 6 à 12,
dans lequel la solution aqueuse administrable est une solution pour perfusion fabriquée par ajout d'au moins un additif à la solution aqueuse administrable, dans lequel l'additif contient au moins un élément sélectionné dans le groupe constitué d'au moins un élément de potassium et de calcium, une solution de glucose à 5 %, un acide aminé et de l'héparine.

14. Procédé de fabrication de la solution aqueuse administrable à un corps vivant selon l'une quelconque des revendications 6 à 13,
dans lequel la solution aqueuse administrable est fabriquée sous forme de solution de culture cellulaire pour cultiver des cellules.

15. Procédé de fabrication de la solution aqueuse administrable à un corps vivant selon l'une quelconque des revendications 6 à 13,
dans lequel la solution aqueuse administrable est fabriquée sous la forme d'une solution aqueuse utilisée par administration ou ingestion orale au corps vivant pour inhiber ou prévenir la prolifération et l'hypertrophie des cancers.
